# EUROPEAN PATENT SPECIFICATION

(11) **EP 1 223 173 B1**
(45) Date of publication and mention of the grant of the patent: **13.07.2005**
(21) Application number: 02076058.3
(22) Date of filing: 20.07.1993
(51) Int. Cl.: C07H 19/04, C07H 21/00, C07H 19/10, C07H 19/20, C07H 19/06, C07H 19/16

(54) **Novel 2'-O-alkyl nucleosides and phosphoramidites processes for the preparation and uses thereof**
Neue 2'-O-Alkyl-Nukleoside und -Phosphoramidite, Verfahren zu ihrer Herstellung und ihre Verwendungen
Nouveaux 2'-O-alkylenucléosides et phosphoramidites, leurs procédés de préparation et leur utilisation

(30) Priority: 23.07.1992 US 918362; 27.10.1992 US 967267; 30.10.1992 US 968849
(43) Date of publication of application: 17.07.2002
(62) Divisional of application: 93917287.0
(73) Proprietor: Isis Pharmaceuticals, Inc., Carlsbad, CA 92008 (US)
(72) Inventor: McGee, Daniel Peter Claude, Vista, California 92083 (US); Cook, Phillip Dan, San Marcos, CA 92069 (US); Guinosso, Charles John, Vista, CA 92083 (US)
(74) Representative: Hallybone, Huw George

(56) References cited:
- DE-A- 4 110 085
- US-A- 5 214 135
- IRIBARREN A M ET AL: "2'-O-ALKYL OLIGORIBONUCLEOTIDES AS ANTISENSE PROBES" PROCEEDINGS OF THE NATIONAL ACADEMY OF SCIENCES OF USA, NATIONAL ACADEMY OF SCIENCE. WASHINGTON, US, vol. 87, 1 October 1990 (1990-10-01), pages 7747-7751, XP002060667 ISSN: 0027-8424
- SPROAT B S ET AL: "NEW SYNTHETIC ROUTES TO SYNTHONS SUITABLE FOR 2'-O-ALLYLOLIGORIBONUCLEOTIDE ASSEMBLY" NUCLEIC ACIDS RESEARCH, OXFORD UNIVERSITY PRESS, SURREY, GB, vol. 19, no. 4, 25 February 1991 (1991-02-25), pages 733-738, XP002060668 ISSN: 0305-1048
- SPROAT B S ET AL: "NEW SYNTHETIC ROUTES TO PROTECTED PURINE 2'-O-METHYLRIBOSIDE-3' -O-PHOSPHORAMIDITES USING A NOVEL ALKYLATION PROCEDURE" NUCLEIC ACIDS RESEARCH, OXFORD UNIVERSITY PRESS, SURREY, GB, vol. 18, no. 1, 1990, pages 41-49, XP002060669 ISSN: 0305-1048
- WAGNER E ET AL: "A SIMPLE PROCEDURE FOR THE PREPARATION OF PROTECTED 2'-O- METHYL OR 2'-O-ETHYL RIBONUCLEOSIDE-3'- O-PHOSPHORAMIDITES" NUCLEIC ACIDS RESEARCH, OXFORD UNIVERSITY PRESS, SURREY, GB, vol. 19, no. 21, 11 November 1991 (1991-11-11), pages 5965-5971, XP000568896 ISSN: 0305-1048
- HIDEO I. ET AL: "SYNTHESIS AND HYBRIDIZATION STUDIES ON TWO COMPLEMENTARY NONA(2'-O-METHYL)RIBONUCLEOTIDES" NUCLEIC ACIDS RESEARCH, OXFORD UNIVERSITY PRESS, SURREY, GB, vol. 15, no. 15, 11 August 1987 (1987-08-11), pages 6131-6148, XP000570336 ISSN: 0305-1048
- ROBINS M.J. ET AL.: "Nucleic acid related compounds. 36. Synthesis of the 2'-O-methyl and 3'-O-methyl ethers of guanosine and 2-aminoadenosine and correlation of O'-methylnucleoside 13C nmr spectral shifts" CANADIAN JOURNAL OF CHEMISTRY., vol. 59, no. 24, 1981, pages 3360-3364, XP002229544 NATIONAL RESEARCH COUNCIL. OTTAWA., CA ISSN: 0008-4042
- COTTEN M. ET AL.: "2O-methyl, 2'-O-ethyl oligoribonucleotides and phosphorothioate oligodeoxyribonucleotides as inhibitors of the in vitro U7 snRNP-dependent mRNA processing event" NUCLEIC ACIDS RESEARCH., vol. 19, no. 10, 1991, pages 2629-2635, XP002229545 OXFORD UNIVERSITY PRESS, SURREY., GB ISSN: 0305-1048

## Description

### FIELD OF INVENTION

This invention is directed to processes for the preparation of 2'-*O*-alkyl guanosine and 2'-*O*-alkyl guanosine analogs, phosphoramidites of these compounds and methods of use thereof.

### BACKGROUND OF THE INVENTION

A number of oligonucleotide analogs have been made. One class of oligonucleotides that have been synthesized are the 2'-*O*-substituted oligonucleotides. Such oligonucleotides have certain unique and useful properties. In United States patent application serial no. 814,961, filed December 24, 1991, entitled Gapped 2' Modified Phosphorothioate Oligonucleotides, assigned to the same assignee as this application, European equivalent published as EP 061 895 A 2' substituted nucleotides are introduced within an oligonucleotide to induce increased binding of the oligonucleotide to a complementary target strand while allowing expression of RNase H activity to destroy the targeted strand.

In a recent article, Sproat, B.S., Beijer, B. and Iribarren, A., *Nucleic Acids Research,* **1990,** *18*:41, the authors noted further use of 2'-*O*-methyl substituted oligonucleotides as "valuable antisense probes for studying pre-mRNA splicing and the structure of spliceosomes".

2'-*O*-methyl and ethyl nucleotides and methods of making the same have been reported by a number of authors.

Robins, M.J., Naik, S.R. and Lee, A.S.K., *J. Org. Chem.*, 39:1891 (1974) reported a low yield synthesis of 2'-*O*-and 3'-*O*-methyl guanosine via a stannous chloride catalyzed monomethylation by diazomethane. As was later reported by Robins, M.J., Hansske, F. and Bernier, S.E., *Can. J. Chem.*, 59:3360 (1981), "convenient and high yield methods have been devised for synthesis of the 2'-*O*- and 3'-*O*-methyl ethers of adenosine, cytidine, and uridine... However, guanosine has presented significant difficulties." In the foregoing paper, the authors reported an improved synthesis of 2'-*O* and 3'-*O*-methyl guanosine. The synthesis was improved by effecting the stannous chloride catalyzed diazomethane methylation of 2,6-diamino-9-(β-*D*-ribofuranosyl)purine (2-aminoadenosine) inplace of guanosine. The diamino purine moiety was then reduced to the corresponding guanine moiety with adenosine deaminase. In a further diazoation reaction described by Singer and Kusmierek, *Biochemistry* 15: 5052 (1976), a mixture of 2' and 3'-O-ethyl guanosine was reported to result from the treatment of guanosine with diazoethane. The alkylation also resulted in alkylation of the heterocyclic base. The alkylated product was treated with base to remove the ethyl group from the heterocyclic base. The resulting product was identified by virtue of having the same UV spectrum as that of guanosine, but a Rf differing from the Rf of guanosine.

A further improvement in the synthesis of 2'-*O*-methyl nucleosides was reported by Inoue, H., Hayase, Y. Imura, A., Iwai, S., Miura, K. and Ohtsuka, E., Nucleic Acids Research, 15:6131 (1987). This method of synthesis was effected utilizing CH₃I in the presence of Ag₂O. This method proved useful for all of the common nucleotides with the exception of guanosine. As reported by these authors, guanosine proved refractory to this synthetic method. Thus these authors again had to effect the 2'-*O*-methylation of guanosine with diazomethane. In order to avoid methylation of the amino functionality of the guanine base moiety, the guanine base moiety was blocked with an isobutyryl group. Additionally, to avoid methyl esterification of the 3'-*O* functionality of the sugar moiety, a TIPDS (tetraisopropyldisiloxane) blocking group was used to block both the 3' and the 5' hydroxyls of the sugar moiety.

Sproat et al., *supra* and Sproat, B.S., Iribarren, A.M., Garcia, R.G. and Beijer, B., *Nucleic Acids Research,* 19:733 (1991) addressed the synthesis of 2'-*O*-methyl guanosine (and 2'-*O*-allyl guanosine). In both of these Sproat et al. publications, the investigators presented a further synthetic pathway to 2'-*O*-methylguanosine and 2'-*O*-allylguanosine. They characterized the further pathway with respect to the prior known synthetic methods as "avoids (ing) ...the use of the highly toxic and potentially explosive reagent diazomethane" and being "far superior to the use of silver oxide/methyl iodide." This same synthetic method of the Sproat et al. investigators is also published in B.S. Sproat and A.I. Lamond, "2'-*O*-Methyloligoribonucleotides: synthesis and applications," *Oligonucleotides and Analogues,* ed. F. Eckstein, (IRL Press, 1991) which described syntheses of 2'-*O*-methylribonucleoside-3'-*O*-phosphoramidites. The uridine phosphoramidite synthesis described therein requires both base and sugar protection of the starting nucleoside prior to alkylation. Only after the base and sugar protecting groups are in place on the uridine is it then alkylated. Post alkylation, the base protecting group is removed followed by 5'-*O*-dimethoxytritylation and phosphitylation. The cytidine phosphoramidite synthesis described by Sproat and Lamond utilizes (and thus requires) the base and sugar blocked 2'-*O*-methyl uridine analog. This analog is then converted to a blocked cytidine analog, the blocking group is removed from the sugar, the analog is dimethoxytritylated and finally phosphitylated. The guanosine phosphoramidite synthesis taught by Sproat and Lamond starts from a 2-amino-6-chloronucleoside having 3' and 5' sugar hydroxy groups blocked. This nucleoside is converted to a 2,6-dichloro derivative. The dichloro compound is then 2'-*O* alkylated. Following *O*-alkylation, the dichloro compound is converted to a diazido intermediate. The diazido intermediate is in turn converted to a diamino intermediate. The diamino intermediate is then deaminated to the guanosine analogue. The 2-amino group of the guanosine analogue is blocked followed by dimethoxytritylation and finally phosphitylation. This guanosine procedure is also published in Sproat, et. al., *Nucleic Acids Research,* **1991** 19:733.

The above synthetic procedures involve multiple steps and numerous reagent treatments - 9 different reagent treatments for uridine, 10 for cytidine and 12 for guanosine. For the cytidine and guanosine compounds at least one of the reagents that is required is not readily available and thus is a very expensive reagent.

Other groups have taught the preparation of other 2'-O-alkylated nucleosides. 2'-*O*-methylthiomethylguanosine, was reported by Hansske, F., Madej, D. and Robins, M.J., *Tetrahedron,* 40:125 (1984). It was produced as a minor byproduct of an oxidization step during the conversion of guanosine to 9-β-*D*-arabinofuranosylguanine, i.e. the arabino analogue of guanosine. The addition of the 2'-*O*-methylthiomethyl moiety is an artifact from the DMSO solvent utilized during the oxidization procedure. The 2'-*O*-methylthiomethyl derivative of 2,6-diaminopurine riboside was also reported in the Hansske et al. publication. It was also obtained as an artifact from the DMSO solvent.

In addition, Gladkaya, et al., *Khim. Prir. Soedin.,* 1989, *4,* 568 discloses N₁-methyl-2'-O-(tetrahydropyran-2-yl) and 2'-O-methyl guanosine. Sproat, et al., *Nucleic Acids Research,* **1991**, *19,* 733 teaches the preparation of 2'-O-allylguanosine. Allylation of guanosine required a further synthetic pathway. Iribarren, et al., *Proc. Natl. Acad. Sci.,* **1990**, *87*, 7747 also studied 2'-O-allyl oligoribonucleotides. Iribarren, et al. incorporated 2'-O-methyl-, 2'-O-allyl-, and 2'-O-dimethylallyl-substitutednucleotides into oligoribonucleotides to study the effect of these RNA analogues on antisense analysis. Iribarren found that 2'-O-allyl containing oligoribonucleotides are resistant to digestion by either RNA or DNA specific nucleases and slightly more resistant to nucleases with dual RNA/DNA specificity, than 2'-O-methyl oligoribonucleotides. However, Iribarren found that 2'-O-dimethylallyl containing oligoribonucleotides exhibited reduced hybridization to complementary RNA sequences as compared to 2'-O-methyl oligoribonucleotides. Thus, Iribarren suggested that further attempts to prepare alkylated RNA probes, especially those superior to 2'-allyl cytidine containing oligoribonucleotides should be limited to 2'-O-alkyl groups containing less than five carbon atoms.

Certain oligonucleotides containing 2'-*O*-alkyl substituted nucleotides are promising candidates for use as human pharmaceuticals. Those having long chain alkyl groups (i.e. four or more carbon atoms) are particularly useful. For example, long chain alkyl groups may accomodate functional groups in appropriate orientation with the opposing strand upon strand hybridization. Thus 2'-*O*- long chain alkyl nucleotides such as 2'-*O*- long chain alkyl guanosine nucleotides are highly desireable in some cases. For use in large scale therapeutic testing and eventually for human pharmaceutical use, large amounts of these oligonucleotides must be synthesized. The large amounts of oligonucleotides in turn requires large amounts of the 2'-*O*-alkyl nucleoside phosphoramidites used in synthesizing the oligonucleotides. Consideration must therefore be given to both cost and purity of the starting phosphoramidites used in the synthesis of such oligonucleotides. As a general premise, as the number of synthetic steps increases, the cost of manufacture increases. Further as the number of steps increases, quality control problems escalate. In view of this, it is evident that there is a great need for new and improved procedures for preparing nucleosides and nucleoside phosphoramidites.

### OBJECTS OF THE INVENTION

It is an object of this invention to provide methods of synthesis of 2'-*O*-alkylated guanosine and guanosine analogues.

It is an object of this invention is to provide new and improved syntheses of 2'-*O*-alkyl guanosine phosphoramidites.

These and other objects will become apparent to persons of ordinary skill in the art from a review of the present specification and appended claims.

### SUMMARY OF THE INVENTION

This invention relates to a process for preparing a 2'-*O*-alkylated guanosine 3'-*O*-phosphoramidite comprising the steps of:
alkylating a 2,6-diamino-9-(ribofuranosyl)purine in the presence of a metal hydride to form a 2,6-diamino-9-(2'-*O*-alkylated ribofuranosyl)purine;
deaminating said 2,6-diamino-9-(2'-*O*-alkylated ribofuranosyl)purine to form a 2'-O-alkylated guanosine;
blocking the 5'-hydroxyl moiety of said 2'-*O*-alkylated guanosine; and
phosphitylating the 3'-position of said 5'-blocked 2'-*O*-alkylated guanosine.

This invention also provides a process for preparing 2'-*O*-substituted guanosine comprising:
treating 2,6-diaminopurine riboside with a base havnig a pK_{b} ≥ pK_{b} of NaH and with a compound having the formula R-L wherein R is an aliphatic or alicyclic group and L is a leaving group, to form 2'-*O*-substituted-2,6-diaminopurine riboside and 3'-*O*-substituted-2,6-diaminopurine riboside wherein said substituent is said aliphatic or alicyclic group;
deaminating said 2'-*O*-substituted-2,6-diaminopurine riboside to yield the said 2'-*O*-substituted guanosine; and
recovering said 2'-*O*-substituted guanosine.

This invention further provides a process for preparing 2'-*O*-alkyl-guanosine comprising:
treating 2,6-diaminopurine riboside with a base having a pK_{b} ≥ pK_{b} of NaH and a compound having the formula R-L wherein R is said alkyl group and L is a leaving group, to form 2'-*O*-alkyl-2,6-diaminopurine riboside;
treating said 2'-*O*-alkyl-2,6-diaminopurine riboside with adenosine deaminase; and
recovering said 2'-*O*-alkyl guanosine.

This invention also provides a process for preparing a 3'-*O*-phosphoramidite of 2'-*O*-alkyl guanosine comprising:
treating 2,6-diaminopurine riboside with a base having a pK_{b} ≥ pK_{b} of NaH and a compound having the formula R-L wherein R is said alkyl group and L is a leaving group, to form 2'-*O*-alkyl-2,6-diaminopurine riboside;
treating said 2'-*O*-alkyl-2,6-diaminopurine riboside with adenosine deaminase to form 2'-*O*-alkyl-guanosine;
protecting the 2-NH₂ moiety of said 2'-*O*-alkyl-guanosine with a protecting group;
protecting the 5'-OH moiety of said 2'-*O*-alkyl-guanosine with a further protecting group;
phosphitylating the 3'-OH moiety of said protected 2'-*O*-alkyl-guanosine; and
recovering said 3'-*O*-phosphoramidite of 2'-*O*-alkyl guanosine.

Except for preparation with diazomethane, heretofore, direct alkylation of guanosine has proven to be refractory. The present invention provides direct 2' and 3' - *O*-alkylation of 2,6-diamino-9-(-β-*D*-ribofuranosyl)purine, i.e. 2,6-diaminopurine riboside or 2-aminoadenosine, which can be effected followed by deamination of the 2'-*O*-alkylated 2,6-diamino purine riboside to the corresponding 2'-*O*-alkylated guanosine. This alkylation can be practiced, if desired, without the use of blocking groups on either the heterocycle or the sugar moieties of the nucleoside. Further unlike the use of diazomethane, which will only yield the methyl alkylation product, alkylation as practiced in this invention is not limited to just methyl alkylation but is used to yield a plenitude of alkyl substituted guanosine and 2,4-diaminopurine riboside compounds. The necessary compounds used in the invention having the formula R-L wherein R is an alkyl group and L is a leaving group, are either commercially available, are known in the literature or can be prepared by procedures analogous to known literature compounds.

The two step alkylation processes of the invention are further distinguished from the six step procedure of the Sproat et al. investigators. See the above-referenced *Nucleic Acids Research,* 18:41 (1990) and *Nucleic Acids Research,* 19:733 (1991) publications. In those procedures 2-amino-6-chloropurine riboside must first be blocked at both the 3' and 5' positions, converted to the 2,6-dichloro derivative, blocked at the 6 purine position, derivatized to the 2'-*O*-methyl or 2'-*O*-allyl derivative, converted to 2,6-diamino derivative, deblocked about the 3' and 5' positions and finally deaminated to the 2'-*O*-methyl or 2'-*O*-allyl guanosine product.

In accordance with the processes of this invention, alkylation is effected directly on 2,6-diamino-9-(β-*D*-ribofuranosyl)purine with an appropriate compound having the formula R-L, wherein R is an alkyl group and L is a leaving group, in the presence of a base having a pKb ≥ pKb of NaH. Alkylation can be limited to mono alkylation by limiting the amount of either the R-L group or the base to a stoichiometric (or equivalent) amount. Alternately dialkylation (on both the 2' and 3' positions) can be practiced by use of an excess R-L group and base to concurrently alkylate both the 2' and the 3' positions.

While not wishing to be bound by theory, it has been observed that alkylation predominates at the 2' position compared to the 3' position. Generally a ratio of from about 7:3 to about 8:2 of 2' to 3' alkylation products are obtained (as determined by TLC). For both TLC as well as preparative scale chromatography, the 2' product generally has a faster Rf than the 3' product. Advantage can be taken of this Rf difference to separate the 2'-*O*- and 3'-*O*- products from each other or from 2'-*O*-,3'-*O*- dialkylated products. Thus the 2' and 3' alkylation products can be separated by procedures such as silica gel chromatography if desired.

For alkyl groups that are generally larger than propyl, further advantage can be taken of the rate of deamination of the 2' product verse the 3' product for separation of the 2'-*O* and 3'-*O* products. Thus mixtures of 2'-*O* and 3'-*O* alkylated 2,6-diamino-9-(β-*D*-ribofuranosyl)purine are subjected to deamination with adenosine deaminase. The enzymatic deamination of the 2'-*O* product is more facile than deamination of the 3'-*O* product. This difference in the rate of deamination allows for separation of the deaminated 2' product, i.e. the 2'-*O*-alkylated guanosine, from the slower or non-deaminated 3' product, i.e. the 2,6-diamino-9-(3'-*O*-alkylated-β-*D*-ribofuranosyl)purine. Additionally procedures such as crystallization has been utilized to further separate a 2' product from the corresponding 3' product by separating the 2'-*O*-alkylated diaminopurine riboside product from the corresponding 3'-*O*-alkylated diaminopurine riboside product.

A preferred base utilized for alkylation is sodium hydride. Other suitable bases may also be utilized, however such bases must have sufficient base strength to remove the proton from the 2' (or 3') hydroxyl moiety of the 2,6-diaminopurine riboside starting material. While not wishing to be bound by theory, generally any base having a pKₐ about 10 pkₐ units greater than the pKₐ of the proton of the 2' hydroxyl moiety of the 2,6-diaminopurine riboside starting material may be used. More specifically, bases having a pK_{b} greater than the pK_{b} of sodium hydride may conveniently be selected. Such bases can be selected from compilations of base such as those given in Table 1, page 220 of March, J. *Advanced Organic Chemistry,* Wiley-Interscience, John Wiley & Sons, New York, 1985.

The alkylation reactions of the invention typically are conducted in DMF as the solvent. Other suitable solvents include DMSO, N-methyl pyrolidone and sulfolone.

Preferably, deamination is effected by use of deaminase enzymes. Particularly preferred is adenosine deaminase. Particularly suitable for use is Adenosine Deaminase Type II available from Sigma Chemical Company, St. Louis, MO. Other deamination reagents may also be employed. The deamination reactions of the invention typically are conducted in a mixture solvent containing an organic solvent and an aqueous buffer. Suitable for use as the organic solvent are DMSO, N-methyl pyrolidone and sulfolone. In preferred embodiments of the present invention deamination is achieved using DMSO as the organic solvent. Suitable for use as the aqueous buffer are buffers having a pH compatible to the pH range of use of the deaminse enzyme. Preferred are phosphate buffers such as sodium phosphate and tris buffers.

In order to enrich the 2' product verse 3' product by elimination of any 3' product, a TIPDS (tetraisopropylsiloxane) protecting group is utilized to protect the 3' and 5' hydroxyl moieties of the sugar portions of the 2,6-diaminopurine riboside. In the same manner, exclusive 3' product would be obtainable by use of a base stable, non-migratory 2'-*O* protecting group. Such base stable, non-migratory protecting groups include but are not limited to tetrahydropyranyl (THP), 4-methoxytetrahydropyran-4-yl (Mthp), 1-[(2-chloro-4-methyl)phenyl-4-methoxypiperidin-4-yl (Ctmp), triphenylmethyl (trityl), mono-, di- and tri-methoxytrityl and other similar protecting groups.

In accordance with this invention there are also provided improved processes for the preparation of 2'-*O*-alkylated guanosine phosphoramidites.

In accordance with methods of the present invention, preparation of a 2'-*O*-alkylated guanosine 3'-*O*-phosphoramidite may comprise the steps of alkylating a 2,6-diamino-9-(ribofuranosyl)purine to form a 2,6-diamino-9-(2'-*O*-alkylated ribofuranosyl)purine; deaminating said 2,6-diamino-9-(2'-*O*-alkylated ribofuranosyl)purine to form a 2'-*O*-alkylated guanosine; blocking the 5'-hydroxyl moiety of said 2'-*O*-alkylated guanosine; and phosphitylating the 3'-position of said 5'-blocked 2'-*O*-alkylated guanosine.

The 3'-*O*-phosphoramidite of 2'-*O*-alkyl guanosine and 2,6-diamino-9-(2'-*O*-alkyl-β-*D*-ribofuranosyl) purine can be provided in some embodiments of the present invention by reaction of 2-NH₂, 5'-OH protected 2'-*O*-alkyl guanosine or 2=NH₂, 6-NH₂, and 5'-OH protected 2,6-diamino-9- (2' -*O*-alkyl-β-*D*-ribofuranosyl) purine with commercially available reagent known to those skilled in the art such as 2-cyanoethyl N,N-diisopropylaminochlorophosphine.

2'-O-alkyl guanosine and 2'-O-alkyl-2,6-diaminopurine riboside may be phosphitylated at the 3'-OH to provide phosphoramidites by methods known in the art such as by protection of the NH₂ moieties (2- or 2- and 6- NH₂, respectively) and 5'-OH moiety followed by reaction with cyanoethyl N,N-diisopropyl aminochlorophosphine.

Compounds of the present invention such as are provided herein can be incorporated into oligomers by procedures known to those skilled in the art.

In the context of this invention, the term "nucleoside" refers to a sugar and a base that are joined together, normally about an "anomeric" carbon on the sugar. Both α and β sugars are encompassed by the present invention. In preferred embodiments of the present invention the nucleoside sugar is a pentofuranosyl sugar, however, other sugars might also be utilized such as carbocyclic or 4'-deoxy-4'-thio sugar analogs.

The term "oligonucleotide" or "oligomer" as used herein, refers to a polynucleotide formed from naturally occuring bases and furanosyl groups joined by native phosphodiester bonds. Oligonucleotides provided will, of course, comprise at least one 2'-O-alkyl guanosine or derivative thereof. Thus, this term effectively refers to naturally occurring species or synthetic species formed from naturally occurring subunits or their close homologs. The term "oligonucleotide" or "oligomer" may also refer to moieties which have portions similar to naturally occurring oligonucleotides but which have non-naturally occurring portions. Thus, oligonucleotides may have altered sugars, altered base moieties, or altered inter-sugar linkages. Exemplary among these are the phosphorothioate and other sulfur-containing species which are known for use in the art.

Further as used in this invention, the term "alkylating" refers to the addition of an alkyl, alkenyl or alkynyl moiety, preferably an alkyl moiety, to the precursors of the nucleosides phosphoramidites of the invention. Alkylation of the 2' position of the nucleoside sugar links the alkylating moiety to the 2' position of the sugar via an ether linkage.

Preferred alkyl moieties include un-substituted and substituted straight chain C₁-C₂₀ alkyl and un-substituted and substituted branch chain C₁-C₂₀ alkyl. Preferred alkenyl groups include un-substituted and substituted straight chain C₂-C₂₀ alkenyl, and un-substituted and substituted branch chain C₂-C₂₀ alkenyl. Preferred alkynyl groups include un-substituted and substituted straight chain C₂-C₂₀ alkynyl and un-substituted and substituted branch chain C₂-C₂₀ alkynyl. Thus preferred alkylation groups include but are not limited to C₁ to C₂₀ straight or branched chain lower alkyl or substituted lower alkyl, C₂ to C₂₀ straight or branched chain lower alkenyl or substituted lower alkynyl, C₂ to C₂₀ straight or branched chain lower alkynyl or substituted lower alkynyl.

Alkyl groups of the invention include but are not limited to C₁-C₂₀ straight and branched chained alkyls such as methyl, ethyl, propyl, butyl, pentyl, hexyl, heptyl, octyl, nonyl, decyl, undecyl, dodecyl, tridecyl, tetradecyl, pentadecyl, hexadecyl, octadecyl, nonadecyl and eicosyl, isopropyl, 2-butyl, isobutyl, 2-methylbutyl, isopentyl, 2-methyl-pentyl, 3-methylpentyl, 2-ethylhexyl and 2-propylpentyl. Alkenyl groups include, but are not limited to, unsaturated moieties derived from the above alkyl groups including, but not limited to, vinyl, allyl and crotyl. Alkynyl groups include unsaturated moieties having at least one triple bond that are derived from the above alkyl groups including, but not limited to, ethynyl and propargyl.

Substituent groups for the above include but are not necessarily limited to halogen (Cl, Br, F), hydroxyl (OH), thiol (SH), keto (C=O), carboxyl (COOH), nitrate (ONO₂), nitro (NO₂), nitroso (NO), nitrile (CN), trifluoromethyl (CF₃), trifluoromethoxy (OCF₃), O-alkyl, S-alkyl, NH-alkyl, N-dialkyl, O-aralkyl, S-aralkyl, NH-aralkyl, amino (NH₂), azido (N₃), hydrazino (NHNH₂), hydroxylamino (ONH₂), isocyanato (OCN), sulfoxide (SO), sulfone (SO₂), sulfide (S-), disulfide (S-S), silyl, heterocyclic, alicyclic, carbocyclic, intercalators, reporter molecules, conjugates, polyamines, polyamides, polyethylene glycols, polyethers, groups that enhance the pharmacodynamic properties of oligonucleotides, and groups that enhance the pharmacokinetic properties of oligonucleotides. Such compounds include 3-penten-2-one, 3-methyl-2-butanol, 2-cyanooctyl, 3-methoxy-4-heptanal, 3-nitrobutyl, 4-isopropoxydodecyl, 4-azido-2-nitrodecyl, 5-mercaptononyl, 4-amino-1-pentenyl as well as other substituted groups. These substituted groups can be introduced in a blocked or protected form and later de-blocked to the parent substituted compound. For example, use of the phthalimido group as a blocked form of an amino substitution is illustrated below.

Other suitable substituent groups include intercalators, reporter groups, reporter enzymes, and conjugates including cholesterols, phospholipids, biotin, phenanthroline, phenazine, phenanthridine, anthraquinone, acridones, pyrenes, stilbenes, oxazolo-pyridocarbazoles, anthraquinones, phenanthridines, phenazines, azidobenzenes, psoralens, porphyrins, cholic acids, folic acids fluoresceins, rhodamines, coumarins, and dyes; steroids, lipophilic molecules, peptides, protein, vitamins, RNA cleaving complexes, metal chelators, alkylators and cross-linking agents.

One particularly preferred substituent group is CF₃. Further particularly preferred substituent groups are phthalimido and imidazole. As noted, use of the phthalimido group allows for introduction of a blocked amino functionality on the alkyl group. Utilizing guanosine analogues prepared in accordance with this invention as intermediates in oligonucleotide synthesis, after oligonucleotide synthesis is complete, the phthalimido group is removed yielding an amino functionality tethered to a guanosine nucleotide within the oligonucleotide sequence. Use of an imidazole moiety as a substituent group on the alkyl functionality introduces the suggested nucleic acid cleaving functionality, imidazole, on a guanosine nucleotide within an oligonucleotide sequence.

Aryl groups include but are not limited to phenyl, tolyl, benzyl, naphthyl, anthracyl, phenanthryl, pyrenyl, and xylyl. Halogens include fluorine, chlorine and bromine. Suitable heterocyclic groups include but are not limited to imidazole, tetrazole, triazole, pyrrolidine, piperidine, piperazine and morpholine. Amines include amines of all of the above alkyl, alkenyl, alkynyl and aryl groups including primary and secondary amines and "masked amines" such as phthalimide. Amines are also meant to include polyalkylamino compounds and aminoalkylamines such as aminopropylamine and further heterocyclo-alkylamines such as imidazol-1, 2 or 4-yl-propylamine. RNA cleaving complexes may be, for example, intercalators or groups which bind in the minor groove of RNA. Intercalators are molecules which insert themselves between neighboring bases of an olignoucleotide. Reporter molecules are molecules which may aid in the identification of a molecule, either visually or otherwise. Cross-linking agents effectively join two groups.

Suitable leaving groups of the present invention include halides such as chloride, bromide, and iodide, sulfonates such as tosyl, brosyl, nosyl, mesyl and trifyl and oxonium ions. In preferred embodiments of the present invention the leaving group is a halide. Still other suitable leaving groups are well known to those skilled in the art.

In accordance with methods of the present invention, the alkylation is preferably conducted in the presence of a base, preferably a metal hydride such as sodium hydride. Alkylation of the 2',3'-*O*-dialkylstannylene derivative of uridine preferably is performed in the presence of a salt such as a metal halide. Cesium flouride and sodium iodide are preferred in some embodiments of the present invention. Additionally, the 5' hydroxyl blocking group is preferably a dimethoxytrityl moiety. The phosphitylating reagent is preferably bis-N,N-diisopropylaminocyanoethylphosphite and the phosphitylating reaction is preferably conducted in the presence of N,N-diisopropylamino-hydrotetrazolide.

In effecting the alkylation of guanosine, 2',6 diaminopurine is alkylated, for example, by methods described in Attorney Docket No. ISIS-710, filed October 27, 1992. The related European application is published as EP 0 651 759.

The amino moiety of the phosphoramidites of the invention can be selected from various amines presently used for such phosphoramidites. Such amines include both aliphatic and heteroaryl amines as are described in various United States patents, principally those to M. Caruthers and associates. These include United States patents 4,668,777, issued May 26, 1987; 4,458,066, issued Jul. 3, 1984; 4,415,732, issued Nov. 15, 1983; and 4,500,707, issued Feb. 19, 1985. One preferred amino group is diisopropylamino.

In addition to the amino moiety of the phosphoramidite, for phosphodiester and phosphorothioate linkages, an additional phosphorous blocking group is used. One preferred blocking group is the cyanoethyl group. Other phosphorous blocking groups include methoxy and 2-(methylsulphonyl)ethyl. Additionally an activating agent is normally used for the phosphoramidite coupling chemistry. One preferred activating agent is N,N-diisopropylaminohydrotetrazolide. Other suitable moieties for these functions are also disclosed in the above noted patents as well as in United States patent 4,725,677, issued Feb. 16, 1988 and Berner, S., Muhlegger, K., and Seliger, H., *Nucleic Acids Research* **1989,** *17*:853; Dahl, B.H., Nielsen, J. and Dahl, O., *Nucleic Acids Research* **1987**, *15*:1729; and Nielson, J. Marugg, J.E., Van Boom, J.H., Honnens, J., Taagaard, M. and Dahl, O., *J. Chem. Research* **1986**, 26.

For use in phosphorothioate linkage, the Beaucage reagent is described in Beaucage, S.L. and Caruthers, M.H., *Tetrahedron Letters* **1981,** *22*:1859 as well as in Zon, G. and Stec, J., Phosphorothioate oligonucleotides: *Oligonucleotides and Analogs A Practical Approach;* Eckstein, F. Ed.; IRL Press, Oxford, 1991, which also describes sulfurization by elemental sulfur.

### EXAMPLES

The following examples illustrate the invention, however, they are not intended as being limiting. In various examples the nomenclature 4,4'-dimethoxytriphenylmethyl and dimethoxytrityl are used interchangeably to reference the DMT blocking group positioned on the 5'-hydroxyl moiety of the various nucleoside and nucleotides of the invention.

NMR spectra were obtained with the following instruments: ¹H-NMR: Varian Gemini-200 (199.975 MHz), ¹³C-NMR: Varian Gemini-200 (50.289 MHz). NMR spectra were recorded using either deuteriochloroform (tetramethylsilane as internal standard) or dimethylsulfoxide-*d*₆ as solvent. The following abbreviations were used to designate the multiplicity of individual signals: s = singlet, d = doublet, t =triplet, q = quartet, ABq = ab quartet, m = multiplet, dd = doublet of doublets, br s = broad singlet. Mass spectra were acquired on a VG 70-SEQ instrument (VG Analytical (Fisons)), using fast atom bombardment ionization (7 kV Xe atoms). Solvent ratios for column chromatography are given as volume/volume. Evaporations of solvents were performed *in vacuo* (60 torr) at 30°C unless otherwise specified. Melting points are reported uncorrected.

### EXAMPLE 1

### 2,6-Diamino-9-(β-D-ribofuranosyl)purine

In accordance with modifications of the procedures described in Robins, M.J., Hanske, F. and Beriner, S.E., *Can. J. Chem.*, 59:3360 (1981), guanosine hydrate (49 g, Aldrich Chemical Co.), toluene (200 ml), hexamethyldisilazane (160 ml, 4.3 eq) and trifluoromethanesulfonic acid (3.7 ml) were loaded in a stainless steel Parr bomb. The bomb was sealed and heated approximately 1/3 submerged in an oil bath at 170° C for 5 days. The bomb was cooled in a dry ice acetone bath and opened. The contents were transferred to a 2 liter round bottom flask using methanol (MeOH) and the solvent evaporated on a Buchii evaporator. 1:1 H₂O/MeOH (600 ml) was added to the residue and the resulting brown suspension was refluxed 4-5 hr. The resulting suspension was evaporated on the Buchii evaporator to remove the methanol (- 1/2 volume). Additional H₂O (≈300 ml) was added and the mixture was heated, treated with charcoal and filtered through a Celite filter pad. Upon cooling, a crystalline solid formed. The solid was isolated by filtration, washed with H₂O and dried under high vacuum at 90° C to yield the product (43.7 g, 89% yield) as a tan solid. UV and NMR spectra of this compound compared to literature values.

This variation of the procedures of Robins, et al. *supra,* eliminated the need to utilize liquid ammonia in the reaction mixture since the ammonia molecule is generated *in situ* from the silazane reagent and the water of hydration of the guanosine hydrate starting material. Further, the use of chlorotrimethylsilane was not necessary nor was it necessary to conduct the reaction under anhydrous conditions, do a preliminary evaporation, or open and re-seal the Parr bomb under a dry nitrogen atmosphere.

### EXAMPLE 2

### 2,6-Diamino-9-(2'-O-propyl-β-D-ribofuranosyl)purine & 2,6-Diamino-9-(3'-O-propyl-β-D-ribofuranosyl)purine

Sodium hydride (NaH) (2.1 g) was added to 2,6-diamino-9-(β-*D*-ribofuranosyl) purine (10.5 g) in dry dimethylformamide (DMF) (150 ml). After stirring for 10 min, iodopropane (6 ml) was added. The solution was stirred for 45 min at room temperature followed by the addition of a further aliquot of NaH (600 mg). The reaction mixture was stirred overnight and then quenched by the addition of ethanol (EtOH) (5 ml). The reaction mixture was evaporated in vacuo, the residue suspended in 10% MeOH/CH₂Cl₂ and purified by silica gel chromatography (300 g) using 5 → 10% MeOH/CH₂Cl₂ as the eluent. The 2',3'-di-*O*-propyl product eluted first followed by the 2'-*O*-propyl product and then the 3'-*O*-propyl product. The 2'-*O*-propyl product containing fractions were pooled and the solvent stripped to yield a crude foam. The foam was crystallized from H₂O (40 ml), washed with cold H₂O and dried to yield 2.9 g of the 2'-*O*-propyl compound. The mother liquor was evaporated, re-chromatographed and crystallized to yield an additional 2.4 g of the 2'-*O*-propyl compound. The second mother liquor was evaporated to yield 4 g of a mixture of 2' and 3'-*O*-propyl compounds as an oil. Fractions containing the 3'-*O*-propyl product as the major product were evaporated and residue crystallized from water. (See Example 17 below for isolation and characterization of the 2',3'-di-*O*-propyl compound).

### 2,6-Diamino-9-(2'-O-propyl-β-D-ribofuranosyl)purine

¹H NMR (DMSO-d₆) δ 0.76 (t, 3, CH₃), 1.4 (tq, 2, CH₂), 3.3 (m, 1, H-5'' + HDO), 3.65-3.45 (m, 3, H-5', O-CH₂), 3.9 (m, 1), 4.25 (br m, 1), 4.38 (dd, 1), 5.1 (br d, 1 3'-OH), 5.45 (br t, 1, 5'-OH), 5.75 (br s, 2, 6-NH₂), 5.83 (d, 1, H-1'), 6.77 (br s, 2, 2-NH₂) and 7.95 (s, 1, H-8). Anal. Calcd. for C₁₃H₂₀N₆O₄·½H₂O: C, 46.91; H, 6.2; N,25.25. Found: C, 47.09; H, 6.37; N, 25.33.

### 2.6-Diamino-9-(3'-O-propyl-β-D-ribofuranosyl)purine

¹H NMR (DMSO-d₆) δ 0.75 (t, 3, CH₃), 1.4 (tq, 2, CH₂), 3.27-3.5 (ABX 2, O-CH₂-), 3.5 and 3.6 (ABX, 2, H-5'), 3.9 (m,1), 4.22 (m, 1), 4.35 (m, 1), 5.1 (br d, 1, 2'-OH), 5.45 (br t, 1, 5'-OH), 5.75 (br s, 2, 6-NH₂), 5.8 (d, 1, H-1'), 6.8 (br s, 2CH₂, 2-H₂) and 7.95 (s, 1, H-8) .

### EXAMPLE 3

### 2'-O-Propylguanosine

A mixture of 2,6-Diamino-9- (2'-*O*-propyl-β-*D*-ribofuranosyl) purine and 2,6-Diamino-9-(3'-*O*-propyl-β-*D*-ribofuranosyl) purine (4.6 gm) and adenosine deaminase (200 mg, Sigma Chemicals Type II) were stirred at room temperature overnight in 0.1 M tris buffer (150 ml, pH 7.4), DMSO (100 ml) and 0.1 M sodium phosphate buffer (10 ml). A further aliquot of adenosine deaminase (140 mg) in 0.1 M phosphate buffer (30 ml) and DMSO (20 ml) was added and the reaction stirred an addition 24 hrs. The solvent was evaporated in vacuo and the residue flash chromatographed on silica gel utilizing 5 → 20% MeOH/CH₂Cl₂. Product-containing fractions were evaporated in vacuo and the residue crystallized from H₂O to yield 2.6 gm of product. m.p. dec > 270° C. ¹H NMR (DMSO-d₆) δ 0.75 (t, 3, CH₃), 1.42 (tq, 2, CH₂), 3.3-3.6 (m, 4, H-5', O-CH₂), 3,85 (m, 1), 4.2 (m, 1), 4.23 (m, 1), 5.10 (t, 1, 5'-OH), 5.13 (d, 1, 3'-OH), 5.75 (d, 1, H-1'), 6.45 (br s, 2, NH₂), 7.95 (s, 1, H-8) and 10.67 (br s, 1, NH). Anal. Calcd. for C₁₃H₁₉N₅O₅: C, 47.99; H, 5.89; N, 21.53. Found: C, 47.90, H, 5.85; N, 21.44.

### EXAMPLE 4

### N2-Isobutyryl-2'-O-propylguanosine

2'-*O*-Propylguanosine (3.6 gm) in pyridine (50 ml) was cooled in an ice bath and trimethylsilyl chloride (8.4 ml, 6 eq.) was added. The reaction mixture was stirred for 30 min and isobutyryl chloride (5.8 ml, 5 eq.) was added. The solution was stirred for 4 hours during which it was allowed to warm to room temperature. The solution was cooled, H₂O added (10 ml) and the solution was stirred for an additional 30 mins. Concentrated NH₄OH (10 ml) was added and the solution evaporated in vacuo. The residue was purified by silica gel chromatography using 10% MeOH/CH₂Cl₂ to elute the product. Product-containing fractions were evaporated to yield 2.5 g of product as a foam. An analytical sample was re-chromatographed on silica and eluted with CH₂Cl₂ → 6% MeOH/CH₂Cl₂. ¹H NMR (DMSO-d₆) δ 0.75 (t, 3, CH₃), 1.13 [d, 6, CH(CH₃)₂], 1.4 (m, 2, CH₂), 2.75 [m, 1, CH(CH₃)₂], 3.52 (m, 6, OCH₂), 3.36 and 3.6 (ABX, 2, H-5'), 3.95 (m, 1), 4.26 (m, 1), 4.33 (m, 1), 5.07 (t, 1, 5'-OH), 5.18 (d, 1, 3'-OH), 5.9 (d, 1, H-1'), 8.25 (s, 1, H-8), 11.65 (br s, 1, NH) and 12.1 (br s, 1, NH). Anal. Calcd. for C₁₇H₂₅N₅O₆·½H₂O: C, 50.49; H, 6.48; N, 17.32. Found: C, 50.81; H, 6.62; N, 17.04.

### EXAMPLE 5

### N2-Isobutyryl-5'-dimethoxytrityl-2'-O-propylguanosine

N2-Isobutyryl-2'-*O*-propylguanosine (2.64 g) was co-evaporated with pyridine and then solubilized in pyridine (180 ml). Dimethoxytrityl chloride (2.4 g, 1.1 eq) and dimethylaminopyridine (50mg) were added with stirring at room temperature. The reaction mixture was stirred overnight and evaporated in vacuo. The residue was partitioned between CH₂Cl₂/ 2x dil Na₂CO₃. The organic phase was dried (MgSO₄) and evaporated. The residue was purified by silica gel chromatography (1:1 EtOAc/Hex → 5% MeOH/EtOAc, 1% TEA) to yield 4.1 g of product. ¹H NMR (DMSO-d₆) δ 0.78 (t, 3, CH₃), 1.12 [d, 6, CH(CH₃)₂], 1.46 (m, 2, CH₂), 2.75 [m, 1, CH(CH₃)₂], 3.35 and 3.55 (ABX, 2, H-5'), 3.73 (s, 6, OCH₂), 4.0 (m, 1), 4.3 (m, 1), 4.4 (m, 1), 5.18 (d, 1, 3'-OH), 5.93 (d, 1, H-1'), 6.8, 7.2, 7.36 (m, 13, DMTr), 8.13 (s, 1, H-8), 11.63 (br s, 1, NH) and 12.1 (br s, 1, NH). Anal. Calcd. for C₃₈H₄₂N₅O₈·H₂O: C, 63.83; H, 6.20; N, 9.80. Found: C, 64.22; H, 6.35; N, 9.55.

### EXAMPLE 6

### N2-Isobutyryl-5'-dimethoxytrityl-2'-O-propylguanosine 3'-β-cyanoethyl-N,N-diisopropylphosphoramidite

A CH₂Cl₂ solution of N2-Isobutyryl-5'-dimethoxytrityl-2'-*O*-propylguanosine (4.1 g), *bis-*(N,N-diisopropylamino)-2-cyanoethylphosphite (3.7 ml, 2 eq) and N,N-diisopropylammonium tetrazolide (0.5 g, 0.5 eq) was stirred at room temperature overnight. The solution was partitioned against dil. Na₂CO₃ and then dil. Na₂CO₃/NaCl and dried over MgSO₄. The solvent was evaporated in vacuo and the residue was purified by silica gel chromatography (120 g, 1%TEA in EtOAc) to yield 5.2 g of product as a foam. ³¹P NMR (CDCl₃) 6 150.5, 150.8.

### EXAMPLE 7

### 2,6-Diamino-9-(2'-O-pentyl-β-D-ribofuranosyl)purine & 2,6-Diamino-9-(3'-O-pentyl-β-D-ribofuranosyl)purine

2,6-Diamino-9-(β-*D*-ribofuranosyl)purine (10 g) was treated with sodium hydride (1.7 g, 1.2 eq) and bromopentane (5.3 ml, 1.2 eq) in DMF (90 ml) as per the procedure of Example 2. Silica gel chromatography yielded three components. The first eluted component (not characterized but believed to be the 2,3-di-(*O*-pentyl) compound was isolated as an oil (700 mg). The next component isolated as a foam (3.3 g) was crystallized from MeOH to yield 2.8 g of 2,6-diamino-9-(2'-*O*-pentyl-β-*D*-ribofuranosyl)purine. The third component isolated as a solid (200 mg) was crystallized from MeOH to yield 80 mg of 2,6-diamino-9-(3'-*O*-pentyl-β-*D*-ribofuranosyl)purine. Fractions containing mixtures of the first and second components were evaporated and the residue crystallized from MeOH to yield a further 900 mg of the 2-*O*-pentyl compound. Further fraction yielded 1.2 g of a mixture of the 2'-*O*-pentyl and 3'-*O*-pentyl compounds.

### 2,6-Diamino-9-(2'-O-pentyl-β-D-ribofuranosyl)purine

¹H NMR (DMSO-d₆) δ 0.75 (t, 3, CH₃), 1.16 (m, 4, CH₂), 1.39 (m, 2, CH₂), 3.53 (m, 2, CH₂), 3.3 and 3.6 (ABX, 2, H-5'), 3.93 (br s, 1), 4.23 (m, 1), 4.38 (m, 1), 5.1 (d, 1 3'-OH), 5.5 (t, 1, 5'-OH), 5.75 (br s, 2, 6-NH₂), 5.82 (d, 1, H-1'), 6.8 (br s, 2, 2-NH₂) and 7.93 (s, 1, H*-*8).

### 2,6-Diamino-9-(3'-O-pentyl-β-D-ribofuranosyl)purine

¹H NMR (DMSO-d₆) δ 0.87 (t, 3, CH₃), 1.3 (m, 4, CH₂), 1.55 (m, 2, CH₂), 3.5 (m, 2, O-CH₂-), 3.6 (m, 2, H-5'), 3.86 (m, 1), 3.95 (m, 1), 4.6 (m, 1), 5.32 (br d, 1 2'-OH), 5.46 (br t, 1, 5'-OH), 5.70 (d, 1, H-1'), 5.75 (br s, 2, 6-NH₂), 6.76 (br s, 2, 2-NH₂) and 7.93 (s, 1, H-8).

### EXAMPLE 8

### 2'-O-Pentylguanosine

2 , 6-diamino-9-(2'-*O*-pentyl-β-*D*-ribofuranosyl)purine (1.9 g) in 0.1 M sodium phosphate buffer (50 ml, pH 6.0) and DMSO (25 ml) was treated with adenosine deaminase (added in two aliquots - first aliquot 50 mg, second aliquot 80 mg) at 35° C as per the procedure of Example 3 to yield 1.4 g of product. ¹H NMR (DMSO-d₆) δ 0.8 (t, 3, CH₃), 1.16 (m, 4, 2x CH₂), 1.4 (m, 2, CH₂), 3.38, 3.6 (m, 4, OCH₂, H-5'), 3.93 (s, 1, H-4'), 4.28 (m, 2, H-2', H-3'), 5.17 (br, 2, 5', 3'-OH), 5.8 (d, 1, H-1'), 6.53 (br s, 2, NH₂), 8.0 (s, 1, H-8) and 10.68 (br, 1, NH).

### EXAMPLE 9

### N2-Isobutyryl-2'-O-pentylguanosine

2'-*O*-pentylguanosine (2.3 g) in pyridine (35 ml) was treated with trimethylsilyl chloride (4.15 ml, 5 eq) and isobutyryl chloride (3.4 ml, 5 eq) as per the procedure of Example 4 to yield the product as a foam (2.3 g). An analytical sample was crystallized from EtOAc/Hex. m.p. 178-180° C. ¹H NMR (DMSO-d₆) δ 0.75 (t, 3, CH₃), 1.1 [m, 10, 2x CH₂, CH(CH₃)₂], 1.4 (m, 2, CH₂), 2.74 [m, 1, CH(CH₃)₂], 3.56 (m, 4, OCH₂, H-5'), 3.93 (m, 1, H-4'), 4.25 (m, 1), 4.34 (m, 1), 5.05 (t, 1, 5'-OH), 5.17 (d, 1, 3'-OH), 5.88 (d, 1, H-1'), 8.27 (s, 1, H-8), 11.65 (br s, 1, NH) and 12.05 (br s, 1, NH). Anal. Calcd. for C₁₉H₂₉N₅O₆: C, 53.89; H, 6.90; N, 16.54. Found: 53.75; H, 6.92; N, 16.40

### EXAMPLE 10

### N2-Isobutyryl-5'-dimethoxytrityl-2'-O-pentylguanosine

N2-Isobutyryl-2'-*O*-pentylguanosine (2.3 g) was treated with dimethoxytrityl chloride (1.7 g, 1.1 eq), and dimethylaminopyridine (100 mg as a catalyst) in pyridine (50 ml) as per the procedure of Example 5 to yield the product as a foam (2.9 g). ¹H NMR (DMSO-d₆) δ 0.83 (t, 3, CH₃), 1.2 [m, 10, 2x CH₂, CH(CH₃)₂], 1.48 (m, 2, CH₂), 2.78 [m, 1, CH(CH₃)₂], 3.4, 3.6 (m, 4, OCH₂, H-5'), 3.75 (s, 6, OCH₃), 4.07 (m, 1), 4.27 (m, 1), 4.42 (m, 1), 5.2 (br d, 1, 3'-OH), 5.95 (d, 1, H-1'), 6.85, 7.25, 7.38 (m, 13, DMTr), 8.15 (s, 1, H-8), 11.67 (br s, 1, NH) and 12.1 (br s, 1, NH). Anal. Calcd. for Anal. Calcd. for C₄₀H₄₇N₅O₈·½H₂O: C, 65.38; H, 6.58; N, 9.53. Found: C, 65.37; H, 6.59; N, 9.39.

### EXAMPLE 11

### N2-Isobutyryl-5'-dimethoxytrityl-2'-O-pentylguanosine 3'-β-cyanoethyl-N,N-diisopropylphosphoramidite

N2-Isobutyryl-5'-dimethoxytrityl-2'-*O*-pentyl-guanosine (1.7 g) was treated with *bis-*(N,N-diisopropylamino)-2-cyanoethyl-phosphite (1.48 g) and N,N-diisopropylammonium tetrazolide (200 mg) as per the procedure of Example 6 to yield the product (1.4 g). ³¹P NMR (CDCl₃) δ 150.5, 150.85.

### EXAMPLE 12

### 2,6-Diamino-9-(2'-O-nonyl-β-D-ribofuranosyl)purine

2,6-Diamino-9-(β-*D*-ribofuranosyl)purine (50 g, 180 mmol) was treated with sodium hydride (8.8 g, 220 mmol) and bromononane (59 g, 54.4 ml, 285 mmol) in DMF (700 ml) as per the procedure of Example 2 (the diamino compound in DMF was cooled in an ice bath during the addition of NaH) to yield 83 g of crude product. 50 g of crude product was purified by silica gel chromatography. Fraction containing 2'-*O*-nonyl and 3'-*O*-nonyl product were combined to give a 77:23 mixture (29 g) of the 2' and 3' product. Pure 2'-*O*-nonyl product is obtained by chromatography. ¹H NMR (DMSO-d₆) δ 0.95 (t, 3, CH₃); 1.17 [m, 12, O-CH₂-CH₂-(CH₂)₆]; 1.42 [m, 2, O-CH₂CH₂(CH₂)₆]; 3.27-3.70 (m, 2, H-5'); 3.50-3.70 [m, 2, O-CH₂(CH₂)₇]; 3.95 (m, 1, H-4'), 4.24 (m, 1, H-3'); 4.40 (m, 1, H-2'); 5.10 (d, 1, 3'-OH, *J*= 5 Hz); 5.50 (t, 1, 5'-OH, *J=* 6 Hz); 5.76 (s, 2, 2-NH₂); 5.83 (d, 1, H-1', *J=* 6.0 Hz); 6.81 (s, 2, 6-NH₂); and 7.96 (s, 1, 8-H).

### EXAMPLE 13

### 2'-O-Nonylguanosine

A mixture of 2,6-diamino-9-(2'-*O*-nonyl-β-*D*-ribofuranosyl)purine and 2,6-diamino-9-(3'-*O*-nonyl-β-*D*-ribofuranosyl)purine (≈ 80:20 mixture, 29 g) in 0.1 M sodium phosphate buffer (50 ml, pH 7.4), 0.1 M tris buffer (1800 ml, pH 7.4) and DMSO (1080 ml) was treated with adenosine deaminase (1.6 g) as per the procedure of Example 3 to yield 60 g of product as an oil. An analytical product was purified by silica gel chromatography and recrystallized from EtOAc. m.p. 258-259° C. ¹H NMR (DMSO-d₆) δ 0.96 (t, 3, CH₃, J= 7 Hz); 1.17 [m, 12, O-CH₂-CH₂-(CH₂)₆]; 1.42 [m, 2, O-CH₂CH₂(CH₂)₆]; 3.27-3.61 (m, 4, H-5', O-CH₂(CH₂)₇]; 3.95 (m, 1, H-4'), 4.10-4.13 (m, 2, H-2', H-3'); 5.13-6.06 (m, 2, 3'-OH 5'-OH); 5.80 (d, 1, H-1', *J*= 6.4 Hz); 6.47 (s, 2, 2-NH₂); 7.98 (s, 1, 8-H) and 10.64 (s, 1, N₁ amide). Anal. Calcd. for C₁₉H₃₁N₅O₅: C, 55.73; H, 7.63; N, 17.10. Found: C, 55.67; H, 7.66; N, 17.02.

### EXAMPLE 14

### N2-Isobutyryl-2'-O-nonylguanosine

2'-*O*-nonylguanosine (14.7 g) in pyridine (360 ml) was treated with trimethylsilyl chloride (23.4 ml) and isobutyryl chloride (30.6 ml) as per the procedure of Example 4 to yield crude product (37 g). The crude material was purified by silica gel chromatography (eluted with 90/10 CHCl₃/MeOH) to yield 14.6 g of product re-crystallized from EtOAc. m.p. 168-169° C. ¹H NMR (DMSO-d₆) δ 0.85 [t, 3, CH₃(nonyl)], 1.14 [m, 18, O-CH₂CH₂(CH₂)₆, CH(CH₃)₂], 1.40 [m, 2, O-CH₂CH₂(CH₂)₆], 2.79 [m, 1, CH(CH₃)₂], 3.31-3.63 (m, 4, H-5', O-CH₂(CH₂)₇]; 3.96 (m, 1, H-4'), 4.27-4.37 (m, 2, H-2', H-3'); 5.10 (t, 1, 5'-OH, *J=* 5 Hz), 5.18 (d, 1, 3'-OH, *J*= 4 Hz), 5.91 (d, 1, H-1', *J=* 6.6 Hz), 8.31 (s, 1, 8-H), 11.73 (s, 1, C₂ amide) and 12.11 (s, 1, N₁ amide). Anal. Calcd. for C₂₃H₃₇N₅O₆: C, 57.60; H, 7.78; N, 14.60. Found: C, 57.63; H, 7.92; N, 14.62.

### EXAMPLE 15

### N2-Isobutyryl-5'-dimethoxytrityl-2'-O-nonylguanosine

N2-Isobutyryl-2'-*O*-nonylguanosine (14 6 g, 30.4 mmol) was treated with dimethoxytrityl chloride (12.1 g, 34 mmol) in pyridine (200 ml) as per the procedure of Example 5 to yield 16 g of purple foam prior to chromatography and 11.5 g after chromatography purification. ¹H NMR (DMSO-d₆) δ 0.84 [t, 3, CH₃(nonyl), *J=* 7 Hz], 1.16 [m, 18, O-CH₂CH₂(CH₂)₆, CH(CH₃)₂], 1.43 [m, 2, O-CH₂CH₂(CH₂)₆], 2.77 [m, 1, CH(CH₃)₂], 3.18-3.63 (m, 4, H-5', O-CH₂(CH₂)₇]; 3.74 (s, 6, DMTr O-CH₃) 4.06 (m, 1, H-4'), 4.27 (m, 1, H-3'); 4.42 (m, 1, H-2'); 5.19 (d, 1, 3'-OH, *J=* 5 Hz), 5.94 (d, 1, H-1', *J=* 5.7 Hz), 6.83-7.38 (m, 13, DMTr aromatic), 8.14 (s, 1, 8-H), 11.65 (s, 1, C₂ amide) and 12.11 (s, 1, N₁ amide). Anal. Calcd. for C₄₄H₅₅N₅O₈: C, 67.59; H, 7.27; N, 8.96. Found: C, 67.59; H, 7.11; N, 8.80.

### EXAMPLE 16

### N2-Isobutyryl-5'-dimethoxytrityl-2'-O-nonylguanosine 3'-β-cyanoethyl-N,N-diisopropylphosphoramidite

N2-Isobutyryl-5'-dimethoxytrityl-2'-*O*-nonylguanosine (2.1 g) was treated with bis-(N,N-diisopropylamino)-2-cyanoethyl-phosphite (1.5 g) and N,N-diisopropylammonium tetrazolide (0.2 g) as per the procedure of Example 6 to yield the product (2.0 g). ³¹P NMR (CDCl₃) δ 150.7 and 150.4 (diastereomers).

### EXAMPLE 17

### 2,6-Diamino-9-(2',3'-di-O-propyl-β-D-ribofuranosyl]purine

The procedure of Example 2 was repeated utilizing 2,6-diamino-9-(β-*D*-ribofuranosyl)purine (10 g), NaH (3 g) and 1-bromopropane (10 ml) in DMF. After evaporation of the reaction solvent, the reaction products were purified by silica gel chromatography. The slower moving component yielded 4.3 g of the 2'-*O*-propyl product as a foam. This foam was crystallized from water to yield 3.6 g of product. The faster moving component isolated as an oil formed crystals upon standing. EtOH was added to the crystals, they were filtered and wash 1 x EtOH to yield 1.1 grams of 2',3'-di-*O*-propyl product. m.p. 165-167° C. ¹H NMR (DMSO-d₆) δ 0.80 and 0.92 (t, 6, CH₃), 1.6 and 1.45 (m, 4, CH₂), 3.7-3.45 (br m, 6), 4.07 (m, 2), 4.5 (dd, 1), 5.55 (br t, 1, 5'-OH), 5.8 (br s, 2, 6-NH₂), 5.85 (d, 1, H-1'), 6.84 (br s, 2, 2-NH₂) and 8.0 (s, 1, H-8).
Anal. Calcd. for C₁₆H₂₆N₆O₄: C, 52.45; H, 7.15; N, 22.94. Found: C, 52.18; H, 7.19; N, 22.75.

### EXAMPLE 18

### N2,N6-Diisobutyryl-2,6-diamiao-9-(2'-O-propyl-β-D-ribofuranosyl)purine

2,6-diamino-9-(2'-*O*-propyl-β-*D*-ribofuranosyl)purine (2.0 g) in pyridine (35 ml) was treated with trimethylsilyl chloride (3.9 ml, 5 eq) and isobutyryl chloride (3.2 ml, 5 eq) as per the procedure of Example 4 to yield a foam after silica gel chromatography. The foam was crystallized from EtOAc/Hex to yield 2.2 g of product. m.p. 140-142° C. ¹H NMR (DMSO-d₆) δ 0.77 (t, 3, CH₃), 1.07, 1.16 [d, 12, 2 x CH(CH₃)₂], 1.5 (m, 2, CH₂), 2.9, 3.03 [m, 2, 2 x CH(CH₃)₂], 3.4 (m, 1, H-5''), 3.58 (m, 3, OCH₂, H-5'), 3.95 (m, 1, H-4'), 4.3 (m, 1), 4.5 (m, 1), 5.02 (t, 1, 5'-OH), 5.2 (d, 1, 3'-OH), 6.03 (d, 1, H-1'), 8.58 (s, 1, H-8), 10.39 (br s, 1, NH), and 10.57 (br s, 1, NH).

### EXAMPLE 19

### N2,N6-Diisobutyryl-2,6-diamino-9-(5'-O-dimethoxytrityl-2'-O-propyl-β-D-ribofuranosyl)purine

N2,N6-Diisobutyryl-2,6-diamino-9-(2'-*O*-propyl-β-*D*-ribo-furanosyl)purine (1.9 g) was treated with dimethoxytrityl chloride (1.5 g, 1.1 eq), and dimethylaminopyridine (20 mg as a catalyst) in pyridine (50 ml) as per the procedure of Example 5 to yield the product as a foam (2.8 g). ¹H NMR (DMSO-d₆) δ 0.79 (t, 3, CH₃), 1.07, 1.16 [d, 12, 2 x CH(CH₃)₂], 1.5 (m, 2, CH₂), 2.9, 3.03 [m, 2, 2 x CH(CH₃)₂], 3.58 (m, 3, OCH₂, H-5'), 4.15 (m, 1, H-4'), 4.4 (m, 1), 4.6 (m, 1), 5.15 (d, 1, 3'-OH), 6.15 (d, 1, H-1'), 6. 8-7.35 (m, 13, DMTr), 8.5 (s, 1, H-8), 10.3 (br s, 1, NH), and 10.57 (br s, 1, NH).

### EXAMPLE 20

### N2,N6-Diisobutyryl-2,6-diamino-9-(5'-O-dimethoxytrityl-2'-O-propyl-β-D-ribofuranosyl)purine 3'-β-cyanoethyl-N,N-diisopropylphosphoramidite

N2,N6-Diisobutyryl-2,6-diamino-9-(5'-*O*-dimethoxytrityl-2'-*O*-propyl-β-*D*-ribofuranosyl)purine (2.6 g) was treated with *bis*-(N,N-diisopropylamino)-2-cyanoethylphosphite (1.7 g) and N,N-diisopropylammonium tetrazolide (300 mg) overnight at room temperature. The reaction mixture was partitioned against dil. Na₂CO₃/CHCl₂ and then Na₂CO₃/NaCl and dried over MgSO₄. The organic layer was evaporated to a foam. The foam was dissolved in CH₂Cl₂ (≈8 ml) and slowly added to Hexanes (500 ml). The solid was filtered and dried to yield the product as a powder (3.1 g). ³¹P NMR (CDCl₃) δ 150.8 and 151.3.

### EXAMPLE 21

### 2,6-Diamino-9-[2'-O-[(N-phthalimido)prop-3-yl]-β-D-ribofuranosyl] purine & 2,6-Diamino-9-[3'-O-[(N-phthalimido) prop-3-yl]-β-D-ribo-furanosyl]purine

2,6-Diamino-9-(β-*D*-ribofuranosyl)purine (14.2 g) was treated with sodium hydride (3 g, 1.5 eq) and N- (3-bromopropyl) phthalimide (5.3 ml, 1.5 eq) in DMF (20 g) at 70° C overnight. The reaction mixture was proportioned between H₂O and Hexanes (1x) and the aqueous layer then extracted 4 x CH₂Cl₂. The organic layer was dried over MgSO₄ and evaporated to a residue. The residue was purified by silica gel chromatography eluted with MeOH/CH₂Cl₂. The 2' -*O*-(N-phthalimido)propyl product eluted first followed by mixed fractions and then the 3'-*O*-(N-phthalimido) product. Evaporations of the fractions gave 3.4 g of the 2'-*O*-(N-phthalimido)propyl product, 3.0 g of mixed 2' and 3' products and 1.4 g of the 3'-*O*-(N-phthalimido)propyl product all as foams. The 3'-*O*-(N-phthalimido)propyl product was crystallized from EtOAc/MeOH to give 270 mg of solid.

### 2,6-Diamino-9-[2'-O-[(N-phthalimido)prop-3-yl]-β-D-ribofuranosyl] purine

¹H NMR (DMSO-d₆) δ 1.8 (tq, 2, -CH₂-), 3.4-3.58 (m, 6, 2x CH₂, H-5'), 3.9 (m, 1), 4.26 (m, 1), 4.37 (m, 1), 5.05 (br d, 1, 3'-OH), 5.4 (br t, 1, 5'-OH), 5.72 (br s, 2, NH₂), 5.8 (br d, 1, H-1'), 6.75 (br s, 2, NH₂), 7.8 (br s, 4, Ar) and 8.93 (s, 1, H-8).

### 2,6-Diamino-9-[3'-O-[(N-phthalimido)prop-3-yl]-β-D-ribofuranosyl] purine

m.p. 220-222° C, ¹H NMR (DMSO-d₆) δ 1.85 (tq, 2, -CH-N), 3.6-3.67 (m, 4, -O-CH₂, H-5'), 3.85 (m, 1), 3.92 (m, 1), 4.6 (m, 1), 5.33 (d, 1, 2'-OH), 5.45 (br t, 1, 5'-OH), 5.65 (d, 1, H-1'), 5.73 (br s, 2, NH₂), 6.75 (br d, 2, NH₂), 7.8-7.85 (m, 4, Ar) and 7.85 (s, 1, H-8). Anal. Calcd. for C₂₁H₂₃N₇O₆: C, 53.73; H, 4.94; N, 20.88. Found: C, 53.59; H, 4.89; N, 20.63.

### EXAMPLE 22

### 2'-O-[(N-Phthalimido)prop-3-yl] guanosine

2,6-diamino-9- [2'-*O*-[(N-phthalimido)prop-3-yl]-β-*D*-ribofuranosyl] purine (3.1 g) in 0.1 M sodium phosphate buffer (3 ml, pH 7.4), 0.05 M tris buffer (65 ml, pH 7.4) and DMSO (45 ml) was treated with adenosine deaminase (200 mg) at room temperature for 5 days as per the procedure of Example 3. The product containing fractions from the silica gel chromatography were evaporated and upon concentration formed white crystals. The crystals were filtered and washed with MeOH to yield 1.1 g of product. An analytical sample was recrystallized from MeOH. m.p. 192-194° C. ¹H NMR (DMSO-d₆) δ 1.82 (m, 2, CH₂), 3.45-3.67 (m, 6, H-5', OCH₂, NCH₂), 3.9 (m, 1), 4.3 (m, 2, H-2', H-3'), 5.1 (m, 2, 5' and 3'-OH), 5.8 (d, 1, H-1'), 6.5 (br s, 2, NH₂), 7.83 (s, 4, phthal), 7.98 (s, 1, H-8) and 10.5 (br s, 1, NH). Anal. Calcd. for C₂₁H₂₂N₆O₇·½H₂O: C, 52.61; H, 4.83; N, 17.53. Found: C, 52.52; H, 4.78; N, 17.38.

### EXAMPLE 23

### N2-Isobutyryl-2'-O-[(N-phthalimido)prop-3-yl] guanosine

2'-*O*-[(N-phthalimido)prop-3-yl] guanosine (7.2 g, crude) in pyridine (35 ml) was treated with trimethylsilyl chloride (11.6 ml, 5 eq) and isobutyryl chloride (8 ml, 5 eq) as per the procedure of Example 4 to yield the product as a crude foam (6.5 g). An analytical sample was obtained by crystallization from EtOAc. m.p. 166-168° C. ¹H NMR (DMSO-d₆) δ 1.15 [d, 6, -CH(CH₃)₂], 1.85 (m, 2, CH₂), 2.8 [m, 1, CH(CH₃)₂], 3.45-3.7 (m, 6, H-5', OCH₂, NCH₂), 3.95 (m, 1), 4.34 (m, 1), 4.4 (m, 1), 5.12 (t, 1, 5'-OH), 5.18 (d, 1, 3'-OH), 5.9 (d, 1, H-1'), 7.83 (s, 4, phthal), 8.3 (s, 1, H-8), 11.65 (br s, 1, NH) and 12.1 (br s, 1, NH). Anal. Calcd. for C₂₅H₂₈N₆O₈·½H₂O: C, 54.64; H, 5.32; N, 15.29. Found: C, 54.46; H, 5.39; N, 14.98.

### EXAMPLE 24

### N2-Isoburyryl-5'-dimethoxytrityl-2'-O-[(N-phthalimido)prop-3-yl]guanosine

N2-Isobutyryl-2'-*O*-[(N-phthalimido)prop-3-yl] guanosine (1.2 g) was treated with dimethoxytrityl chloride (820 mg, 1.1 eq), and dimethylaminopyridine (20 mg as a catalyst) in pyridine (50 ml) as per the procedure of Example 5 utilizing 1:1 Hex/EtOAc, then EtOAc then 5%MeOH/EtOAc with 1% TEA as eluent. The product containing fraction were evaporated to yield the product as a foam (1.7 g). ¹H NMR (DMSO-d₆) δ 1.1 [d, 6, -CH(CH₃)₂], 1.85 (m, 2, CH₂), 2.75 [m, 1, CH(CH₃)₂], 3.45-3.7 (m, 6, H-5', OCH₂, NCH₂), 3.75 (s, 6, OCH₃), 4.0 (m, 1), 4.32 (m, 1), 4.4 (m, 1), 5.2 (d, 1, 3'-OH), 5.93 (d, 1, H-1'), 6.83, 7.2, 7.35 (m, 13, DMTr), 7.78 (s, 4, phthal), 8.15 (s, 1, H-8), 11.6 (br s, 1, NH) and 12.05 (br s, 1, NH). Anal. Calcd. for C₄₆H₄₆N₆O₁₀·H₂O: C, 64.18; H, 5.62; N, 9.76. Found: C, 64.42; H, 5.78; N, 9.53.

### EXAMPLE 25

### N2-Isobutyryl-5'-dimethoxytrityl-2'-O-[(N-phthalimido)prop-3-yl]guanosine 3'-β-cyanoethyl-N,N-diisopropylphosphoramidite

N2-Isobutyryl-5'-dimethoxytrityl-2'-*O*-[(N-phthalimido) prop-3-yl] guanosine (1.6 g) was treated with *bis*-(N,N-diisopropylamino)-2-cyanoethylphosphite (1.48 g) and N,N-diisopropylammonium tetrazolide (200 mg) as per the procedure of Example 6 to yield the product (2.0 g). ³¹P NMR (CDCl₃) δ 150.9.

### EXAMPLE 26

### N2-Dimethylaminomethylidene-5'-dimethoxytrityl-2'-O-[(N-phthalimido)prop-3-yl] guanosine

2'-*O*-[(N-phthalimido)prop-3-yl] guanosine (900 mg) in DMF (20 ml) was treated with N,N-dimethylformamide dimethyl acetal (2 ml). The reaction mixture was stirred for 2 hr and evaporated under high vac at 52° C. The residue was co-evaporated 1 x with pyridine and taken up in solution in pyridine. Dimethoxytrityl chloride (713 mg, 1.1 eq) and dimethylaminopyridine (20 mg as a catalyst) were added. The reaction mixture was stirred overnight, partitioned between Na₂CO₃/CH₂Cl₂, dried over MgSO₄ and purified by silica gel chromatography as per the procedure of Example 5 to yield 1.7 g of product as an off white solid. ¹H NMR (DMSO-d₆) δ 1.88 (m, 2, CH₂), 3.1 [d, 6, N=CHN(CH₃)₂], 3.3 (m, 2, H-5'), 3.67 (m, 4, OCH₂, NC₂), 3.78 (s, 6, 2x OCH₃), 4.0 (m, 1, H-4'), 4.35 (m, 2, H-2', H-3'), 5.2 (d, 1, 3'-OH), 5.95 (d, 1, H-1'), 6.85, 7.25, 7.39 (m, 13, DMTr), 7.85 (s, 4, phthal), 7.95 [s, 1, H-8), 8.5 (s, 1, N=CHN(CH₃)₂] and 11.39 (s, 1, NH₂). Anal. Calcd. for C₄₅H₄₅N₇O₉·½H₂O: C, 64.58; H, 5.54; N, 11.71. Found: C, 64.10; H, 5.65; N, 11.47.

### EXAMPLE 27

### N2-Dimethylaminomethylidene-5'-dimethoxytrityl-2'-O-[(N-phthalimido)prop-3-yl] guanosine 3'-β-cyanoethyl-N,N-diisopropylphosphoramidite

N2-dimethylaminomethylidene-5'-dimethoxytrityl-2'-*O*-[(N-phthalimido)prop-3-yl] guanosine (1.7 g), *bis*-(N,N-diisopropylamino)-2-cyanoethylphosphite (1.4 ml) and N,N-diisopropylammonium tetrazolide (170 mg) were stirred overnight at room temperature. The reaction mixture was partitioned between CH₂Cl₂ and Na₂CO₃ (2 x). The organic phase was dried over MgSO₄ and evaporated to an oil. The oil was dissolved in a minimum of CH₂Cl₂ and added dropwise to ≈ 900 ml Hexanes to precipitate the product. The solid was isolated and dried to yield 2.1 g of product. ¹P NMR (CDCl₃) δ 150.4, 150.6.

### EXAMPLE 28

### 2,6-Diamino-9-[2'-O-(N-phthalimido)pent-5-yl]-β-D-ribofuranosyl] purine

2,6-Diamino-(9-β-*D*-ribofuranosyl)purine (6.7 g) was treated with sodium hydride (1.3 g) and N-(5-bromopentyl) phthalimide (7.8 g, 1.1 eq) in DMF (60 ml) at room temperature for three days. The reaction mixture was proportioned between H₂O and CH₂Cl₂ and extracted 4 x CH₂Cl₂. The combined organic layers were dried over MgSO₄ and evaporated. The residue was purified by silica gel chromatography eluted with 5 → 10% MeOH/CH₂Cl₂. The 2'-*O*-(N-phthalimido)pentyl containing fractions were collected and evaporated to a yellow foam to give 2.2 g of product. An analytical sample was crystallized from EtOH. m.p. 173-175° C. ¹H NMR (DMSO-d₆) δ 1.2 (m, 2, - CH₂-), 1.47 (m, 4, 2x CH₂), 3.55, 3.65 (m, 6, O-CH₂, H-5', NCH₂), 3.95 (m, 1), 4.28 (m, 1), 4.4 (m, 1), 5.13 (d, 1, 3'-OH), 5.5 (t, 1, 5'-OH), 5.77 (br s, 2, 6-NH₂), 5.84 (br d, 1, H-1'), 6.8 (br s, 2, 2-NH₂), 7.86 (M, 4, phthal) and 7.95 (s, 1, H-8). Anal. Calcd. for C₂₃H₂₇N₇O₆: C, 55.50; H, 5.47; N, 19.71. Found: C, 55.44; H, 5.51; N, 19.30.

### EXAMPLE 29

### 2'-O-[(N-Phthalimido)pent-5-yl] guanosine

A mixture of the 2,6-diamino-9-[2' -*O*-[(N-phthalimido) pent-5-yl]-β-*D*-ribofuranosyl]purine and 2,6-diamino-9-[3'-*O*-[(N-phthalimido) pent-5-yl]-β-*D*-ribofuranosyl] purine isomers (2.2 g) in 0.1 M tris buffer (60 ml, pH 7.4), 0.1 M NaPO₄ buffer (2 ml, pH 7.4) and DMSO (40 ml) was treated with adenosine deaminase (60 mg) at room temperature for 5 days as per the procedure of Example 3. The product containing fractions from the silica gel chromatography were evaporated to give the product (1.0 g) as a crude white solid. An analytical sample was prepared by the addition of MeOH to form crystals. m.p. 178-180° C. ¹H NMR (DMSO-d₆) δ 1.24 (m, 2, CH₂), 1.5 (m, 4, 2x CH₂), 3.5-3.6 (m, 6, H-5' , OCH₂, NCH₂), 3.87 (m, 1, H-4'), 4.25 (m, 2, H-2', H-3'), 5.1 (m, 2, 5' and 3'-OH), 5.78 (d, 1, H-1'), 6.5 (br s, 2, NH₂), 7.84 (M, 4, phthal), 7.98 (s, 1, H-8) and 10.67 (br s, 1, NH). Anal. Calcd. for C₂₃H₂₆N₆O₇·½H₂O: C, 54.43; H, 5.36; N, 16.56. Found: C, 54.79; H, 5.24; N, 16.61.

### EXAMPLE 30

### N2-Isobutyryl-2'-O-[(N-phthalimido)pent-5-yl] guanosine

2'-*O*-[(N-phthalimido)pent-5-yl] guanosine (1.6 g, crude) in pyridine (35 ml) was treated with trimethylsilyl chloride (2.0 ml, 5 eq) and isobutyryl chloride (1.68 ml, 5 eq) as per the procedure of Example 4 to yield the product as a foam. This foam was co-evaporated 2 x with EtOAc followed by the addition of EtOAc and heating to yield white crystals (950 mg). m.p. 202-204° C. ¹H NMR (DMSO-d₆) δ 1.1 [d, 6, - CH(CH₃)₂], 1.17 (m, 2, CH₂), 1.43 (m, 4, 2x CH₂), 2.74 [m, 1, CH(CH₃)₂], 3.45-3.55 (m, 6, H-5', OCH₂, NCH₂), 3.9 (m, 1), 4.25 (m, 1), 4.3 (m, 1), 5.07 (t, 1, 5'-OH), 5.15 (d, 1, 3'-OH), 5.87 (d, 1, H-1'), 7.8 (s, 4, phthal), 8.27 (s, 1, H-8), 11.67 (br s, 1, NH) and 12.06 (br s, 1, NH). Anal. Calcd. for C₂₇H₃₂N₆O₈·½H₂O: C, 56.14; H, 5.76; N, 14.55. Found: C, 56.45; H, 5.74; N, 14.41.

### EXAMPLE 31

### N2-Isobutyryl-5'-dimethoxytrityl-2'-O-[(N-phthalimido)pent-5-yl] guanosine

N2-Isobutyryl-2'-*O*-[(N-phthalimido)pent-5-yl] guanosine (0.95 g) was treated with dimethoxytrityl chloride (620 mg, 1.1 eq), and dimethylaminopyridine (20 mg as a catalyst) in pyridine (50 ml) as per the procedure of Example 5 utilizing EtOAc 1% TEA and then 5% MeOH EtOAc/CH₂Cl₂ with 1% TEA as eluent. The product containing fractions were evaporated to yield the product as a foam (1.4 g). H NMR (DMSO-d₆) δ 1.14 [d, 6, -CH(CH₃)₂], 1.25 (m, 2, CH₂), 1.53 (m, 4, 2x CH₂), 2.77 [m, 1, CH(CH₃)₂], 3.3-3.6 (m, 6, H-5', OCH₂, NCH₂), 3.75 (s, 6, OCH₃), 4.07 (m, 1), 4.33 (m, 1), 4.4 (m, 1), 5.18 (d, 1, 3'-OH), 5.94 (d, 1, H-1'), 6.83, 7.2, 7.53 (m, 13, DMTr), 7.8 (s, 4, phthal), 8.15 (s, 1, H-8), 11.6 (br s, 1, NH) and 12.1 (br s, 1, NH). Anal. Calcd. for C₄₈H₅₀N₆O₁₀·½H₂O: C, 65.52; H, 5.84; N, 9.55. Found: C, 65.55; H, 5.94; N, 9.20.

### EXAMPLE 32

### 2,6-Diamiao-9-[3',5'-O-(tetraisopropyldisiloxane-1,3-diyl)-β-D-ribofuranosyl]purine

To a suspension of 2,6-diamino-9-(β-*D*-ribofuranosyl)purine (10.5 g) in pyridine (100 ml) was added 1,3-dichlorotetraisopropyldisiloxane (TIPDS, 12.6 g). The reaction was stirred at room temperature for 4 hours and an additional 1.3 g of 1,3-dichlorotetraisopropyldisiloxane was added followed by stirring overnight. The reaction mixture was poured into ice water and the insoluble product (11.6 g) collected by filtration. An analytical sample was recrystallized from EtOAc/Hexanes. m.p. 170-172° C. Anal. Calcd. for C₂₂H₄₀N₆O₅Si₂·½H₂O: C, 49.5; H, 7.74; N, 15.7. Found: 49.57; H, 7.82; N, 15.59.

### EXAMPLE 33

### 2,6-Diamino-9-[3',5'-O-(tetraisopropyldisiloxane-1,3-diyl)-2-O-methyl-β-D-ribofuranosyl]purine

A mixture of 2,6-Diamino-9-[3,5-*O*-(tetraisopropyldisiloxane-1,3-diyl)-β-*D*-ribofuranosyl]purine (8.8 g) in DMF (120 ml) and methyl iodide (3 ml, 3 eq) was cooled in an ice bath and NaH (60% in oil, 1.0 g, 1.5 eq) added. After 20 min the reaction was quenched with MeOH and partitioned between sat. NH₄Cl and CH₂Cl₂. The organic phase was washed with 1 x NH₄Cl, dried over MgSO₄ and evaporated. The residue was crystallized from hot EtOH/H₂O to yield the product (8.5 g) as crystals. m.p. 87-89°C. ¹H NMR (DMSO-d₆) δ 1.05 (m, 28, TIPDS), 3.57 (s, 3, OCH₃), 3.98 (m, 1, H-4'), 3.92 and 4.07 (ABX, 2, H-5'), 4.13 (d, 1), 4.6 (dd, 1, H-3'), 5.76 (br s, 2, NH₂), 5.8 (s, 1, H-1'), 6.77 (br s, 2, NH₂) AND 7.77 (s, 1 H-8).

### EXAMPLE 34

### 2,6-Diamino-9-(2'-O-methyl-β-D-ribofuranosyl)purine

To a solution of 2,6-Diamino-9-[3',5'-*O*-(tetraisopropyldisiloxane-1,3-diyl)-2'-*O*-methyl-β-*D*-ribofuranosyl]purine (8.5 g) in THF (50 ml) was added 1M tetrabutylammonium fluoride in THF (Aldrich, 20 ml). The reaction mixture was stirred for 2 hrs and filtered. The filter cake was washed with 2 x EtOAc and air dried to give 4.0 g of crude product. An analytical sample was crystallized from hot MeOH. m.p. 133-135° C. ¹H NMR (DMSO-d₆) δ 3.3 (s, 3, OCH₃), 3.58 (m, 2, H-5'), 3.98 (m, 1, H-4'), 4.28 (m, 2, H-2', H-3'), 5.23 (br s, 1, 3'-OH), 5.48 (br t, 1, 5'-OH), 5.77 (br s, 2, NH₂), 5.82 (d, 1, H-1'), 6.83 (br s, 2, NH₂) and 7.95 (s, 1, H-8). Anal. Calcd. for C₁₁H₁₆N₆O₄·½H₂O: C, 43.28; H, 5.61; N, 27.52. Found: C, 43.51; H, 5.62; N, 27.26.

### EXAMPLE 35

### 2'-O-Methylguanosine

2,6-Diamino-9-(2'-*O*-methyl-β-*D*-ribofuranosyl)purine (9.5 g) in 0.1M sodium phosphate buffer (200 ml, pH 7.4) and DMSO (25 ml) was treated with adenosine deaminase (Type II Sigma) at RT for 4 days. The resulting suspension was cooled and filtered and the resulting filter cake washed with H₂O and dried to a white solid (4.0 g). The solid was recrystallized from hot H₂O to yield 2.9 g of product. m.p. 236-238° C. ¹H NMR (DMSO-d₆) δ 3.3 (s, 3, OCH₃), 3.53 and 3.6 (ABX, 2, H-5'), 3.87 (m, 1, H-4'), 4.15 (m, 1, H-2'), 4.25 (m, 1, H-3'), 5.13 (t, 1, 5'-OH), 5.23 (d, 1, 3'-OH), 5.8 (d, 1, H-1'), 6.48 (br s, 2, NH₂), 7.96 (s, 1, H-8) and 10.68 (br s, 1, NH). Anal. Calcd. for C₁₁H₁₅N₅O₅·½H₂O: C, 43.14; H, 5.26; N, 22.86. Found: C, 43.59; H, 5.34; N, 23.04.

### EXAMPLE 36

### N2-Isobutyryl-2'-O-methylguanosine

2'-*O*-methylguanosine (3.5 g) in pyridine (100 ml) was treated with trimethylsilyl chloride (9 ml, 6 eq) and isobutyryl chloride (6.2 ml) at RT for 4 hr. The reaction mixture was cooled in an ice bath, H₂O (20 ml) was added and stirring continued for an additional 20 min. NH₄OH (20 ml) was added and after stirring for 30 min the reaction mixture was evaporated. The residue was triturated with H₂O, filtered and the filtrate evaporated and purified by silica gel chromatography as per the procedure of Example 4 to yield the product as an off white solid (1.5 g). ¹H NMR (DMSO-d₆) δ 1.1 [d, 6, CH(CH₃)₂], 2.77 [m, 1, CH(CH₃)₂], 3.33-3.6 (m, 5, OCH₃, H-5'), 3.93 (m, 1, H-4'), 4.22 (m, 1), 4.3 (m, 1), 5.1 (t, 1, 5'-OH), 5.28 (d, 1, 3'-OH), 5.9 (d, 1, H-1'), 8.28 (s, 1, H-8) and 11.9 (br s, 1, NH).

### EXAMPLE 37

### N2-Isobutyryl-5'-dimethoxytrityl-2'-O-methylguanosine

N2-Isobutyryl-2'-*O*-methylguanosine (1.5 g) was treated with dimethoxytrityl chloride (1.5 g, 1.1 eq), and dimethylaminopyridine (100 mg as a catalyst) in pyridine (50 ml) as per the procedure of Example 5 to yield the product as a foam (2.6 g). ¹H NMR (DMSO-d₆) δ 1.14 (d, 6, CH(CH₃)₂], 2.75 [m, 1, CH(CH₃)₂], 3.5 (m, 2, H-5'), 3.74 (s, 6, OCH₃), 4.05 (m, 1), 4.33 (m, 1), 5.26 (d, 1, 3'-OH), 5.95 (d, 1, H-1'), 6.83, 7.2, 7.35 (m, 13, DMTr), 8.15 (s, 1, H-8), 11.6 (br s, 1, NH) and 12.1 (br s, 1, NH).

### EXAMPLE 38

### N2-Isobutyryl-5'-dimethoxytrityl-2'-O-methylguanosine 3'-β-cyanoethyl-N,N-diisopropylphosphoramidite

N2-Isobutyryl-5'-dimethoxytrityl-2'-*O*-methylguanosine (20 g) was treated with *bis*-(N,N-diisopropylamino)-2-cyanoethylphosphite (10.8 g) and N,N-diisopropylammonium tetrazolide (1.6 g) as per the procedure of Example 6 to yield the product (15.7 g). ³¹P NMR (CDCl₃) δ 148.97 and 147.96.

### EXAMPLE 39

### N2,N6-Diisobutyryl-2,6-diamino-9-(2'-O-methyl-β-D-ribofuranosyl) purine

2,6-diamino-9-(2'-*O*-methyl-β-*D*-ribofuranosyl)purine (700 mg) in pyridine (20 ml) was treated with trimethylsilyl chloride (2.1 ml, 7 eq) and isobutyryl chloride (1.25 ml, 5 eq) as per the procedure of Example 4 to yield the product as a foam (900 mg) after silica gel chromatography.

### EXAMPLE 40

### N2,N6-Diisobutyryl-2,6-diamino-9-(5'-O-dimethoxytrityl-2'-O-methyl-β-D-ribofuranosyl)purine

N2,N6-Diisobutyryl-2,6-diamino-9-(2'-*O*-methyl-β-*D*-ribofuranosyl)purine (900 mg) was treated with dimethoxytrityl chloride (1.0 g) and dimethylaminopyridine (20 mg as a catalyst) in pyridine (30 m) as per the procedure of Example 5 to yield the product as a foam (700 mg). ¹H NMR (DMSO-d₆) 6 0.96-1.16 [m, 12, 2x CH(CH₃)₂], 2.9 and 3.05 [M, 2, 2x CH(CH₃)₂], 3.18 and 3.37 (ABX, 2, H-5'), 3.38 (s, 3, OCH₃), 3.7 (s, 6, OCH₃), 4.05 (m, 1, H-4'), 4.44 (m, 2, H-2',H-3'), 5.24 (d, 1, 3'-OH), 6.06 (d, 1, H-1'), 6.78, 7.2, 7.33 (m, 13, Ar), 8.22 (s, 1, H-8) , 10.3 (br s, 1, NH) and 10.57 (br s, 1, NH).

### EXAMPLE 41

### N2,N6-Diisobutyryl-2,6-diamino-9-(5'-O-dimethoxytrityl-2'-O-methyl-β-D-ribofuranosyl)purine 3'-β-cyanoethyl-N,N-diisopropylphosphoramidite

N2,N6-Diisobutyryl-2,6-diamino-9-(5'-*O*-dimethoxytrityl-2'-*O*-methyl-β-*D*-ribofuranosyl)purine (600 mg) was treated with *bis-*(N,N-diisopropylamino)-2-cyanoethylphosphite (500 µl) and N,N-diisopropylammonium tetrazolide (80 mg) overnight at RT. The reaction mixture was partitioned against dil. Na₂CO₃/CHCl₂ and then Na₂CO₃/NaCl and dried over MgSO₄. The organic layer was evaporated to a foam (500 mg). ³¹P NMR (CDCl₃) δ 151.1 (doublet).

### EXAMPLE 42

### 2,6-Diamiao-9-(2'-O-octadecyl-β-D-ribofuranosyl)purine

2,6-Diamino-9-(β-D-ribofuranosyl)purine (50 g, 180 mmol) and sodium hydride (7 g) in DMF (1 l) were heated to boiling for 2 hr. Iodooctadecane (100 g) was added at 150° C and the reaction mixture allowed to cool to RT. The reaction mixture was stirred for 11 days at RT. The solvent was evaporated and the residue purified by silica gel chromatography. The product was eluted with 5% MeOH/CH₂Cl₂. The product containing fraction were evaporated to yield the product (11 g). ¹H NMR (DMSO-d₆) δ 0.84 (t, 3, CH₂); 1.22 [m, 32, O-CH₂-CH₂-(CH₂)₁₆-]; 1.86 (m, 2, O-CH₂CH₂-); 3.25 (m, 2, O-CH₂-); 3.93 (d, 1, 4'H), 4.25 (m, 1, 3'H); 4.38 (t, 1, 2'H); 5.08 (d, 1, 3'-OH); 5.48 (t, 1, 5'-OH); 5.75 (s, 2, 6-NH₂); 5.84 (d, 1, 1'-H); 6.8 (s, 2, 2-NH₂); and 7.95 (s, 1, 8-H).

### EXAMPLE 43

### 2'-O-Octadecylguanosine

2,6-Diamino-9-(2'-*O*-octadecyl-β-*D*-ribofuranosyl) purine (10 g) in 0.1 M sodium phosphate buffer (50 ml, pH 7.4), 0.1 M tris buffer (1000 ml, pH 7.4) and DMSO (1000 ml) was treated with adenosine deaminase (1.5 g) as per the procedure of Example 3. At day 3, day 5 and day 7 an additional aliquot (500 mg, 880 mg and 200 mg, respectively) of adenosine deaminase was added. The reaction was stirred for a total of 9 day and after purification by silica gel chromatography yielded the product (2 g). An analytical sample was recrystallized from MeOH ¹H NMR (DMSO-d₆) δ 0.84 (t, 3, CH₃), 1.22 [s, 32, O-CH₂-CH₂-(CH₂)₁₆], 5.07 (m, 2, 3'-OH 5'-OH); 5.78 (d, 1, 1'-H); 6.43 (s, 2, NH₂), 7.97 (s, 1, 8-H) and 10.64 (s, 1, NH₂). Anal. Calcd. for C₂₈H₄₉N₅O₅: C, 62.80; H, 9.16; N, 12.95. Found: C, 62.54; H, 9.18; N, 12.95.

### EXAMPLE 44

### N2-Isobutyryl-2'-O-octadecylguanosine

2'-*O*-Octadecylguanosine (1.9 g) in pyridine (150 ml) was treated with trimethylsilyl chloride (2 g, 5 eq) and isobutyryl chloride (2 g, 5 eq) as per the procedure of Example 4. The product was purified by silica gel chromatography (eluted with 3% MeOH/EtOAc) to yield 1.2 g of product. ¹H NMR (DMSO-d₆) δ 0.85 [t, 3, CH₃], 1.15 [m, 38, O-CH₂CH₂(CH₂)₁₆, CH(CH₃)₂], 2.77 [m, 1, CH(CH₃)₂], 4.25 (m, 2, 2'H, 3'H); 5.08 (t, 1, 5'-OH), 5.12 (d, 1, 3'-OH), 5.87 (d, 1, 1'-H), 8.27 (s, 1, 8-H), 11.68 (s, 1, NH₂) and 12.08 (s, 1, NH₂). Anal. Calcd. for C₃₂H₅₅N₅O₆: C, 63.47; H, 9.09; N, 11.57. Found: C, 63.53; H, 9.20; N, 11.52.

### EXAMPLE 45

### 2,6-Diamino-9-[2'-O-(imidazol-1-yl)butyl-β-D-ribofuranosyl] purine

2,6-Diamino-(9-β-*D*-ribofuranosyl)purine (5.0 g) in DMF (400 ml) was treated with sodium hydride (0.78 g). After stirring an additional 30 min a further portion of sodium hydride (2.6 g) was added immediately followed by bromobutylimidazole (9.9 g) in DMF (25 ml). The reaction mixture was stirred overnight and quenched with H₂O. The reaction mixture was filtered through celite and evaporated to yield an oily product. TLC showed a mixture of isomers.

### EXAMPLE 46

### 2'-O-(Imidazol-1-yl)butylguanosine

A mixture of the 2,6-diamino-9-[2'-*O*-(imidazol-1-yl)butyl-β-*D*-ribofuranosyl]purine and 2,6-diamino-9-[3'-*O*-(imidazol-1-yl)butyl-β-*D*-ribofuranosyl]purine isomers in 0.1 M tris buffer (pH 7.4), 0.1 M NaSO₄ buffer (pH 7.4) and DMSO is treated with adenosine deaminase at RT for 5 days as per the procedure of Example 3. The product containing fractions are purified by silica gel chromatography and the product containing fraction evaporated to give the product.

### EXAMPLE 47

### N2-Isobutyryl-2'-O-(imidazol-1-yl)butylguanosine

2'-*O*-(imidazol-1-yl)butylguanosine in pyridine will be treated with trimethylsilyl chloride (5 eq) and isobutyryl chloride (5 eq) as per the procedure of Example 4 to yield the product.

### EXAMPLE 48

### N2-Isobutyryl-5'-dimethoxytrityl-2'-O-(imidazol-1-yl)butylguanosine

N2-Isobutyryl-2'-*O*-(imidazol-1-yl)butylguanosinewill be treated with dimethoxytrityl chloride (1.1 eq), and dimethylaminopyridine (as a catalyst) in pyridine as per the procedure of Example 5. After chromatography purification, the product containing fractions will be evaporated to yield the product).

### EXAMPLE 86

### N2,N6-Diisobutyrylamino-9-(2'-O-methyl-β-D-ribofuranosyl)purine

N2,N6-Diamino-9-(2'-*O*-methyl-β-*D*-ribofuranosyl)purine (1.6 g, 5.39 mmol, see Example 34) was co-evaporated with pyridine (25 ml). A suspension of the residue in pyridine (40 ml) was cooled in an ice bath and trimethylsilyl chloride (4.8 ml) was added. The reaction mixture was stirred for 30 mins followed by the addition of butyryl chloride (2.8 ml, 5 eq). The resulting reaction mixture was stirred at room temperature for 4 hours. H₂O (10 ml) and conc. NH₄OH (10 ml) were added with stirring to quench the reaction mixture. After 30 mins, the reaction mixture was evaporated and the residue purified on a silica gel column using CH₂Cl₂ → 10% MeOH/CH₂Cl₂ to elute the product. The appropriate fractions were evaporated to yield the product as an oil (2.4 g).

### EXAMPLE 87

### N2,N6-Diisobutyrylamiao-9-(5'-O-dimethoxytrityl-2'-O-methyl-β-D-ribofuranosyl)purine

N2,N6-Diisobutyrylamino-9-(2'-*O*-methyl-β-*D*-ribofuranosyl)purine (2.4 g) was co-evaporated with pyridine and redissolved in pyridine. Dimethoxytrityl chloride (1.8 g, 1 eq) and dimethylaminopyridine (5 mg) were added and the resulting solution was stirred overnight at room temperature. The solvent was partly evaporated and the residue partition between CH₂Cl₂ - dil. Na₂CO₃. The organic phase was washed with dil. Na₂CO₃, dried with MgSO₄ and evaporated. The residue was purified on a silica gel column eluted with Hexanes/EtOAc (1:1) containing 1% triethylamine. The fraction contain the product were evaporated to yield the product as a foam (2.4 g).

### EXAMPLE 88

### N2,N6-Diisobutyrylamino-9-[5'-O-dimethoxytrityl-2'-O-methyl-3'-O-(β-cyanoethyl N,N-diisopropylphosphoramide)-β-D-ribofuranosyl]purine

N2,N6-Diisobutyrylamino-9-(5'-*O*-dimethoxytrityl-2'-*O*-methyl-β-*D*-ribofuranosyl)purine (1.7 g, 0.0023 mol) was treated with bis-N,N-diisopropylaminocyanoethylphosphite (1.48 ml, 2 eq.) and N,N-diisopropylaminohydrotetrazolide (200 mg) at room temperature overnight. The reaction mixture was partitioned between dil. Na₂CO₃/CH₂Cl₂, the organic phase was dried over MgSO₄ and evaporated. The residue was loaded on a silica gel column and eluted with Hexanes/EtOAc (3:1 → 1:1 with 1% triethylamine) to give the product as a solid foam (1.73 g). ³¹P-NMR (CD₃CN, H₃PO₄ std.) shows the diastereomers.

## Claims

1. A process for preparing a 2'-*O*-alkylated guanosine 3'-*O*-phosphoramidite comprising the steps of:
alkylating a 2,6-diamino-9-(ribofuranosyl)purine in the presence of a metal hydride to form a 2,6-diamino-9-(2'-*O*-alkylated ribofuranosyl)purine;
deaminating said 2,6-diamino-9-(2'-*O*-alkylated ribofuranosyl)purine to form a 2'-*O*-alkylated guanosine;
blocking the 5'-hydroxyl moiety of said 2'-*O*-alkylated guanosine; and
phosphitylating the 3'-position of said 5'-blocked 2'-*O*-alkylated guanosine.

2. The process of claim 1, wherein the 2,6-diamino-9-(ribofuranosyl)purine is alkylated with an alkylating agent X-L, wherein X is R₁-(R₂)ₙ; R₁ is C₁-C₂₀ alkyl, C₂-C₂₀ alkenyl or C₂-C₂₀ alkynyl; R₂ is halogen, hydroxyl, thiol, keto, carboxyl, nitro, nitroso, nitrile, trifluoromethyl, trifluoromethoxy, O-alkyl, S-alkyl, NH-alkyl, N-dialkyl, O-aryl, S-aryl, NH-aryl, O-aralkyl, S-aralkyl, NH-aralkyl, amino, N-phthalimido, imidazole, azido, hydrazino, hydroxylamino, isocyanato, sulfoxide, sulfone, sulfide, disulfide, silyl, aryl, heterocycle, carbocycle, intercalator, reporter molecule, conjugate, polyamine, polyamide, polyalkylene glycol, or polyether; n is an integer from 0 to 6; and L is a leaving group; in the presence of a metal hydride to form a 2,6-diamino-9-(2'-*O*-alkylated ribofuranosyl)purine.

3. The process of claim 1 or 2, wherein said blocking step adds a dimethoxytrityl group.

4. The process of claim 1 or 2, wherein said phosphitylation step is conducted with bis-N,N-diisopropylaminocyanoethylphosphite.

5. The process of claim 4, wherein said phosphitylation step is conducted in the presence of N,N-diisopropylaminohydrotetrazolide.

6. The process of claim 1 or 2, further comprising blocking the N2-amino moiety of said 2'-*O*-alkylated guanosine prior to blocking said 5'-hydroxyl moiety.

7. The process of claim 1 or 2, wherein said deamination step is performed using adenosine deaminase.

8. A process for preparing 2'-*O*-substituted guanosine comprising:
treating 2,6-diaminopurine riboside with a base having a pK_{b} ≥ pK_{b} of NaH and with a compound having the formula R-L wherein R is an aliphatic or alicyclic group and L is a leaving group, to form 2'-*O*-substituted-2,6-diaminopurine riboside and 3'-*O*-substituted-2,6-diaminopurine riboside wherein said substituent is said aliphatic or alicyclic group;
deaminating said 2'-*O*-substituted-2,6-diaminopurine riboside to yield the said 2'-*O*-substituted guanosine; and
recovering said 2'-*O*-substituted guanosine.

9. The process of claim 8, wherein said aliphatic or alicyclic group is selected from the group consisting of C₁-C₂₀ straight or branched alkyl, alkenyl, or alkynyl chain, or C₁-C₂₀ alicyclic, respectively.

10. The process of claim 9, wherein the substituent of said C₁-C₂₀ substituted straight or branched alkyl, alkenyl, or alkynyl chain, or C₁-C₂₀ substituted alicyclic is halogen, hydroxyl, thiol, keto, carboxyl, nitrates, nitro, nitroso, nitrile, trifluoromethyl, trifluoromethoxy, O-alkyl, S-alkyl, NH-alkyl, N-dialkyl, O-aralkyl, S-aralkyl, NH-aralkyl, amino, azido, hydrazino, hydroxylamino, isocyanato, sulfoxide, sulfone, sulfide, disulfide, silyl, heterocyclic, alicyclic, carbocyclic, intercalators, reporter molecules, conjugates, polyamines, polyamides, polyethylene glycols, and polyethers.

11. The process of claim 8, wherein the 2,6-diaminopurine riboside is treated with an alkylating agent X-L, wherein X is R₁-(R₂)ₙ; R₁ is C₁-C₂₀ alkyl, C₂-C₂₀ alkenyl or C₂-C₂₀ alkynyl; R₂ is halogen, hydroxyl, thiol, keto, carboxyl, nitro, nitroso, nitrile, trifluoromethyl, trifluoromethoxy, O-alkyl, S-alkyl, NH-alkyl, N-dialkyl, O-aryl, S-aryl, NH-aryl, O-aralkyl, S-aralkyl, NH-aralkyl, amino, N-phthalimido, imidazole, azido, hydrazino, hydroxylamino, isocyanato, sulfoxide, sulfone, sulfide, disulfide, silyl, aryl, heterocycle, carbocycle, intercalator, reporter molecule, conjugate, polyamine, polyamide, polyalkylene glycol, or polyether; n is an integer from 0 to 6; and L is a leaving group; to form 2'-*O*-substituted-2,6-diaminopurine riboside and 3'-*O*-substituted-2,6-diaminopurine riboside wherein said substituent is said group X.

12. The process of claim 8 or 11, further comprising:
separating said 2'-*O*-substituted-2,6-diaminopurine riboside from said 3'-*O*-substituted-2,6-diaminopurine riboside.

13. The process of claim 12, wherein said 2'-*O*-substituted-2,6-diaminopurine riboside is separated from said 3'-*O*-substituted-2,6-diaminopurine riboside by conducting said deamination under conditions such that the rate of deamination of said 2'-*O*-substituted-2,6-diaminopurine riboside is accelerated compared to the rate of deamination of said 3'-*O*-substituted-2,6-diaminopurine riboside.

14. The process of claim 13, wherein said accelerated rate of deamination of said 2'-*O*-substituted-2,6-diaminopurine riboside is achieved by use of the enzyme adenosine deaminase.

15. The process of claim 8 or 11, wherein said 2'-*O*-substituted-2,6-diaminopurine is deaminated with adenosine deaminase.

16. The process of claim 8 or 11, wherein said base is sodium hydride.

17. The process of claim 8 or 11, wherein said 2'-*O*-substituted-2,6-diaminopurine riboside is physically separated from said 3'-*O*-substituted-2,6-diaminopurine riboside by crystallization or chromatography.

18. A process for preparing 2'-*O*-alkyl-guanosine comprising:
treating 2,6-diaminopurine riboside with a base having a pK_{b} ≥ pK_{b} of NaH and a compound having the formula R-L wherein R is said alkyl group and L is a leaving group, to form 2'-*O*-alkyl-2,6-diaminopurine riboside;
treating said 2'-*O*-alkyl-2,6-diaminopurine riboside with adenosine deaminase; and
recovering said 2'-*O*-alkyl guanosine.

19. The process of claim 18, wherein said alkyl group is a C₁-C₂₀ straight chain or branched chain, saturated or unsaturated alkyl or a C₁-C₂₀ straight chain or branched chain, saturated or unsaturated, substituted alkyl.

20. The process of claim 19, wherein said alkyl group is C₁-C₂₀ saturated, straight chain alkyl or C₁-C₂₀ saturated, straight chain substituted alkyl.

21. The process of claim 19, wherein the substituent of said C₁-C₂₀ straight chain or branched chain, saturated or unsaturated, substituted alkyl is halogen, hydroxyl, thiol, keto, carboxyl, nitrates, nitro, nitroso, nitrile, trifluoromethyl, trifluoromethoxy, O-alkyl, S-alkyl, NH-alkyl, N-dialkyl, O-aralkyl, S-aralkyl, NH-aralkyl, amino, azido, hydrazino, hydroxylamino, isocyanato, sulfoxide, sulfone, sulfide, disulfide, silyl, heterocyclic, alicyclic, carbocyclic, intercalators, reporter molecules, conjugates, polyamines, polyamides, polyethylene glycols, and polyethers.

22. The process of claim 18, wherein the 2,6-diaminopurine riboside is treated with an alkylating agent X-L, wherein X is R₁-(R₂)ₙ; R₁ is C₁-C₂₀ alkyl, C₂-C₂₀ alkenyl or C₂-C₂₀ alkynyl; R₂ is halogen, hydroxyl, thiol, keto, carboxyl, nitro, nitroso, nitrile, trifluoromethyl, trifluoromethoxy, O-alkyl, S-alkyl, NH-alkyl, N-dialkyl, O-aryl, S-aryl, NH-aryl, O-aralkyl, S-aralkyl, NH-aralkyl, amino, N-phthalimido, imidazole, azido, hydrazino, hydroxylamino, isocyanato, sulfoxide, sulfone, sulfide, disulfide, silyl, aryl, heterocycle, carbocycle, intercalator, reporter molecule, conjugate, polyamine, polyamide, polyalkylene glycol, or polyether; n is an integer from 0 to 6; and L is a leaving group; to form 2'-*O*-alkyl-2,6-diaminopurine riboside.

23. The process of claim 18 or 22, wherein said base is sodium hydride.

24. The process of claim 18 or 22, further comprising:
separating said 2'-*O*-alkyl-2,6-diaminopurine riboside from further alkylated products resulting from said treatment of said 2,6-diaminopurine riboside with said base and said compound R-L or said alkylating agent X-L respectively.

25. A process for preparing a 3'-*O*-phosphoramidite of 2'-*O*-alkyl guanosine comprising:
treating 2,6-diaminopurine riboside with a base having a pK_{b} ≥ pK_{b} of NaH and a compound having the formula R-L wherein R is said alkyl group and L is a leaving group, to form 2'-*O*-alkyl-2,6-diaminopurine riboside;
treating said 2'-*O*-alkyl-2,6-diaminopurine riboside with adenosine deaminase to form 2'-*O*-alkyl-guanosine;
protecting the 2-NH₂ moiety of said 2'-*O*-alkyl-guanosine with a protecting group;
protecting the 5'-OH moiety of said 2'-*O*-alkyl-guanosine with a further protecting group;
phosphitylating the 3'-OH moiety of said protected 2'-*O*-alkyl-guanosine; and
recovering said 3'-*O*-phosphoramidite of 2'-*O*-alkyl guanosine.

26. The process of claim 25, wherein the 2,6-diaminopurine riboside is treated with an alkylating agent X-L, wherein X is R₁-(R₂)ₙ; R₁ is C₁-C₂₀ alkyl, C₂-C₂₀ alkenyl or C₂-C₂₀ alkynyl; R₂ is halogen, hydroxyl, thiol, keto, carboxyl, nitro, nitroso, nitrile, trifluoromethyl, trifluoromethoxy, O-alkyl, S-alkyl, NH-alkyl, N-dialkyl, O-aryl, S-aryl, NH-aryl, O-aralkyl, S-aralkyl, NH-aralkyl, amino, N-phthalimido, imidazole, azido, hydrazino, hydroxylamino, isocyanato, sulfoxide, sulfone, sulfide, disulfide, silyl, aryl, heterocycle, carbocycle, intercalator, reporter molecule, conjugate, polyamine, polyamide, polyalkylene glycol, or polyether; n is an integer from 0 to 6; and L is a leaving group; to form 2'-*O*-alkyl-2,6-diaminopurine riboside.

27. The process of claim 25 or 26, wherein said protected 2'-*O*-alkyl guanosine is phosphitylated with 2-cyanoethyl N,N-diisopropylaminochlorophosphine.

28. The process of claim 25 or 26, wherein said base is sodium hydride.

## Patentansprüche

1. Verfahren zur Herstellung eines 2'-O-alkylierten Guanosin-3'-O-phosphoramidits, umfassend die Schritte:
das Alkylieren eines 2,6-Diamino-9-(ribofuranosyl)purins in der Gegenwart eines Metallhydrids unter Bildung eines 2,6-Diamino-9-(2'-O-alkylierten Ribofuranosyl)purins,
das Deaminieren des 2,6-Diamino-9-(2'-O-alkylierten Ribofuranosyl)purins unter Bildung eines 2'-O-alkylierten Guanosins,
das Blockieren der 5'-Hydroxyleinheit des 2'-O-alkylierten Guanosins und
das Phosphitylieren der 3'-Position des 5'-blockierten 2'-O-alkylierten Guanosins.

2. Verfahren nach Anspruch 1, wobei das 2,6-Diamino-9-(ribofuranosyl)purin mit einem Alkylierungsmittel X-L, wobei X R₁-(R₂)ₙ ist, R₁ C₁-C₂₀ Alkyl, C₂-C₂₀ Alkenyl oder C₂-C₂₀ Alkinyl ist, R₂ ein Halogen, Hydroxyl, Thiol, Keto, Carboxyl, Nitro, Nitroso, Nitril, Trifluormethyl, Trifluormethoxy, O-Alkyl, S-Alkyl, NH-Alkyl, N-Dialkyl, O-Aryl, S-Aryl, NH-Aryl, O-Aralkyl, S-Aralkyl, NH-Aralkyl, Amino, N-Phthalimido, Imidazol, Azido, Hydrazino, Hydroxylamino, Isocyanato, Sulfoxid, Sulfon, Sulfid, Disulfid, Silyl, Aryl, Heterocyclus, Carbocyclus, Interkalator, Reportermolekül, Konjugat, Polyamin, Polyamid, Polyalkylenglykol oder Polyether ist, n eine ganze Zahl von 0 bis 6 ist und L eine Abgangsgruppe ist, in der Gegenwart eines Metallhydrids unter Bildung eines 2,6-Diamino-9-(2'-O-alkylierten Ribofuranosyl)purins alkyliert wird.

3. Verfahren nach Anspruch 1 oder 2, wobei der Blockierungsschritt eine Dimethoxytritylgruppe zugibt.

4. Verfahren nach Anspruch 1 oder 2, wobei der Phosphitylierungsschritt mit Bis-N,N-diisopropylaminocyanoethylphosphit durchgeführt wird.

5. Verfahren nach Anspruch 4, wobei der Phosphitylierungsschritt in der Gegenwart von N,N-Diisopropylaminohydrotetrazolid durchgeführt wird.

6. Verfahren nach Anspruch 1 oder 2, weiter umfassend das Blockieren der N2-Aminoeinheit von dem 2'-O-alkylierten Guanosin vor dem Blockieren der 5'-Hydroxyleinheit.

7. Verfahren nach Anspruch 1 oder 2, wobei der Deaminierungsschritt unter Verwendung von Adenosindeaminase durchgeführt wird.

8. Verfahren zum Herstellen von 2'-O-substituiertem Guanosin, umfassend:
das Behandeln von 2,6-Diaminopurinribosid mit einer Base mit einem pK_{b} ≥ pK_{b} von NaH und mit einer Verbindung mit der Formel R-L, wobei R eine aliphatische oder alicyclische Gruppe ist und L eine Abgangsgruppe ist, unter Bildung von 2'-O-substituiertem 2,6-Diaminopurinribosid und 3'-O-substituiertem 2,6-Diaminopurinribosid, wobei der Substituent die aliphatische oder alicyclische Gruppe ist,
das Deaminieren des 2'-O-substituierten 2,6-Diaminopurinribosids, um das 2'-O-substituierte Guanosin zu ergeben, und
das Rückgewinnen des 2'-O-substituierten Guanosins.

9. Verfahren nach Anspruch 8, wobei die aliphatische oder alicyclische Gruppe aus der Gruppe, bestehend aus C₁-C₂₀ geradkettiger oder verzweigtkettiger Alkyl-, Alkenyl- oder Alkinylkette bzw. C₁-C₂₀ alicyclischen, ausgewählt ist.

10. Verfahren nach Anspruch 9, wobei der Substituent der C₁-C₂₀ substituierten geradkettigen oder verzweigtkettigen Alkyl-, Alkenyl- oder Alkinylkette oder C₁-C₂₀ substituierten alicyclischen Halogen, Hydroxyl, Thiol, Keto, Carboxyl, Nitrate, Nitro, Nitroso, Nitril, Trifluormethyl, Trifluormethoxy, O-Alkyl, S-Alkyl, NH-Alkyl, N-Dialkyl, O-Aralkyl, S-Aralkyl, NH-Aralkyl, Amino, Azido, Hydrazino, Hydroxylamino, Isocyanato, Sulfoxid, Sulfon, Sulfid, Disulfid, Silyl, Heterocyclus, Alicyclus, Carbocyclus, Interkalatoren, Reportermoleküle, Konjugate, Polyamine, Polyamide, Polyethylenglykole und Polyether ist.

11. Verfahren nach Anspruch 8, wobei das 2,6-Diaminopurinribosid mit einem Alkylierungsmittel X-L, wobei X R₁-(R₂)ₙ ist, R₁ C₁-C₂₀ Alkyl, C₂-C₂₀ Alkenyl oder C₂-C₂₀ Alkinyl ist, R₂ Halogen, Hydroxyl, Thiol, Keto, Carboxyl, Nitro, Nitroso, Nitril, Trifluormethyl, Trifluormethoxy, O-Alkyl, S-Alkyl, NH-Alkyl, N-Dialkyl, O-Aryl, S-Aryl, NH-Aryl, O-Aralkyl, S-Aralkyl, NH-Aralkyl, Amino, N-Phthalimido, Imidazol, Azido, Hydrazino, Hydroxylamino, Isocyanato, Sulfoxid, Sulfon, Sulfid, Disulfid, Silyl, Aryl, Heterocyclus, Carbocyclus, Interkalator, Reportermolekül, Konjugat, Polyamin, Polyamid, Polyalkylenglykol oder Polyether ist, n eine ganze Zahl von 0 bis 6 ist und L eine Abgangsgruppe ist, unter Bildung von 2'-O-substituiertem-2,6-Diaminopurinribosid und 3'-O-substituiertem-2,6-Diaminopurinribosid, wobei der Substituent die Gruppe X ist, behandelt wird.

12. Verfahren nach Anspruch 8 oder 11, weiter umfassend:
das Abtrennen des 2'-O-substituierten-2,6-Diaminopurinribosids von dem 3'-O-substituierten-2,6-Diaminopurinribosid.

13. Verfahren nach Anspruch 12, wobei das 2'-O-substituierte-2,6-Diaminopurinribosid von dem 3'-O-substituierten-2,6-Diaminopurinribosid durch Durchführen der Deaminierung unter Bedingungen derart, daß die Deaminierungsrate des 2'-O-substituierten-2,6-Diaminopurinribosids im Vergleich zu der Deaminierungsrate des 3'-O-substituierten-2,6-Diaminopurinribosids beschleunigt ist, abgetrennt wird.

14. Verfahren nach Anspruch 13, wobei die beschleunigte Deaminierungsrate des 2'-O-substituierten-2,6-Diaminopurinribosids durch Verwendung des Enzyms Adenosindeaminase erreicht wird.

15. Verfahren nach Anspruch 8 oder 11, wobei das 2'-O-substituierte-2,6-Diaminopurin mit Adenosindeaminase deaminiert wird.

16. Verfahren nach Anspruch 8 oder 11, wobei die Base Natriumhydrid ist.

17. Verfahren nach Anspruch 8 oder 11, wobei das 2'-O-substituierte-2,6-Diaminopurinribosid physikalisch von dem 3'-O-substituierten-2,6-Diaminopurinribosid durch Kristallisation oder Chromatographie abgetrennt wird.

18. Verfahren zur Herstellung von 2'-O-Alkyl-guanosin, umfassend:
das Behandeln von 2,6-Diaminopurinribosid mit einer Base mit einem pK_{b} ≥ pK_{b} von NaH und einer Verbindung mit der Formel R-L, wobei R die Alkylgruppe ist und L eine Abgangsgruppe ist, unter Bildung von 2'-O-Alkyl-2,6-diaminopurinribosid,
das Behandeln des 2'-O-Alkyl-2,6-diaminopurinribosids mit Adenosindeaminase und
das Rückgewinnen des 2'-O-Alkyl-guanosins.

19. Verfahren nach Anspruch 18, wobei die Alkylgruppe ein C₁-C₂₀ geradkettiges oder verzweigtkettiges, gesättigtes oder ungesättigtes Alkyl oder ein C₁-C₂₀ geradkettiges oder verzweigtkettiges, gesättigtes oder ungesättigtes, substituiertes Alkyl ist.

20. Verfahren nach Anspruch 19, wobei die Alkylgruppe C₁-C₂₀ gesättigtes, geradkettiges Alkyl oder C₁-C₂₀ gesättigtes, geradkettiges substituiertes Alkyl ist.

21. Verfahren nach Anspruch 19, wobei der Substituent von dem C₁-C₂₀ geradkettigen oder verzweigtkettigen, gesättigten oder ungesättigten, substituierten Alkyl Halogen, Hydroxyl, Thiol, Keto, Carboxyl, Nitrate, Nitro, Nitroso, Nitril, Trifluormethyl, Trifluormethoxy, O-Alkyl, S-Alkyl, NH-Alkyl, N-Dialkyl, O-Aralkyl, S-Aralkyl, NH-Aralkyl, Amino-Azido, Hydrazino, Hydroxylamino. Isocyanato, Sulfoxid, Sulfon, Sulfid, Disulfid, Silyl, Heterocyclus, Alicyclus, Carbocyclus, Interkalatoren, Reportermoleküle, Konjugate, Polyamine, Polyamide, Polyethyleneglykole und Polyether ist.

22. Verfahren nach Anspruch 18, wobei das 2,6-Diaminopurinribosid mit einem Alkylierungsmittel X-L, wobei X R₁-(R₂)ₙ ist, R₁ C₁-C₂₀ Alkyl, C₂-C₂₀ Alkenyl oder C₂-C₂₀ Alkinyl ist, R₂ Halogen, Hydroxyl, Thiol, Keto, Carboxyl, Nitro, Nitroso, Nitril, Trifluoromethyl, Trifluoromethoxy, O-Alkyl, S-Alkyl, NH-Alkyl, N-Dialkyl, O-Aryl, S-Aryl, NH-Aryl, O-Aralkyl, S-Aralkyl, NH-Aralakyl, Amino, N-Phthalimido, Imidazol, Azido, Hydrazino, Hydroxylamino, Isocyanato, Sulfoxid, Sulfon, Sulfid, Disulfid, Silyl, Aryl, Heterocyclus, Carbocyclus, Interkalator, Reportermolekül, Konjugat, Polyamin, Polyamid, Polyalkylenglykol oder Polyether ist, n eine ganze Zahl von 0 bis 6 ist und L eine Abgangsgruppe ist, unter Bildung von 2'-O-Alkyl-2,6-diaminopurinribosid behandelt wird.

23. Verfahren nach Anspruch 18 oder 22, wobei die Base Natriumhydrid ist.

24. Verfahren nach Anspruch 18 oder 22, weiter umfassend:
das Abtrennen des 2'-O-Alkyl-2,6-diaminopurinribosids von weiter alkylierten Produkten, die aus der Behandlung des 2,6-Diaminopurinribosids mit der Base und der Verbindung R-L bzw. dem Alkylierungsmittel X-L resultieren.

25. Verfahren zur Herstellung eines 3'-O-Phosphoramidits von 2'-O-Alkyl-guanosin, umfassend:
das Behandeln von 2,6-Diaminopurinribosid mit einer Base mit einem pK_{b} ≥ pK_{b} von NaH und einer Verbindung mit der Formel R-L, wobei R die Alkylgruppe ist und L eine Abgangsgruppe ist, unter Bildung von 2'-O-Alkyl-2,6-diaminopurinribosid,
das Behandeln des 2'-O-Alkyl-2,6-diaminopurinribosids mit Adenosindeaminase unter Bildung von 2'-O-Alkyl-guanosin,
das Schützen der 2-NH₂-Einheit des 2'-O-Alkyl-guanosins mit einer Schutzgruppe,
das Schützen der 5'-OH-Einheit des 2'-O-Alkyl-guanosins mit einer Schutzgruppe,
das Phosphitylieren der 3'-OH-Einheit des geschützten 2'-O-Alkyl-guanosins, und
das Rückgewinnen des 3'-O-Phosphoramidits von 2'-O-Alkyl-guanosin.

26. Verfahren nach Anspruch 25, wobei das 2,6-Diaminopurinribosid mit einem Alkylierungsmittel X-L, wobei X R₁-(R₂)ₙ ist, R₁ C₁-C₂₀ Alkyl, C₂-C₂₀ Alkenyl oder C₂-C₂₀ Alkinyl ist, R₂ Halogen, Hydroxyl, Thiol, Keto, Carboxyl, Nitro, Nitroso, Nitril, Trifluoromethyl, Trifluoromethoxy, O-Alkyl, S-Alkyl, NH-Alkyl, N-Dialkyl, O-Aryl, S-Aryl, NH-Aryl, O-Aralkyl, S-Aralkyl, NH-Aralkyl, Amino, N-Phthalimido, Imidazol, Azido, Hydrazino, Hydroxylamino, Isocyanato, Sulfoxid, Sulfon, Sulfid, Disulfid, Silyl, Aryl, Heterocyclus, Carbocyclus, Interkalator, Reportermolekül, Konjugat, Polyamin, Polyamid, Polyalkylenglykol oder Polyether ist, n ein ganze Zahl von 0 bis 6 ist und L eine Abgangsgruppe ist, unter Bildung von 2'-O-Alkyl-2,6-diaminopurinribosid behandelt wird.

27. Verfahren nach Anspruch 25 oder 26, wobei das geschützte 2'-O-Alkyl-guanosin mit 2-Cyanoethyl-N,N-diisopropylaminochlorphosphin phosphityliert wird.

28. Verfahren nach Anspruch 25 oder 26, wobei die Base Natriumhydrid ist.

## Revendications

1. Procédé de préparation d'un guanosine 3'-O-phosphoramidite 2'-O-alkylé comprenant les étapes consistant à :
alkyler une 2,6-diamino-9-(ribofuranosyl)purine en présence d'un hydrure métallique pour former une 2,6-diamino-9-(ribofurasonyl 2'-O-alkylé)purine ;
désaminer ladite 2,6-diamino-9-(ribofuranosyl 2'-O-alkylé)purine pour former une guanosine 2'-O-alkylée ;
bloquer la fraction 5'-hydroxyle de ladite guanosine 2'-O-alkylée ; et
phosphityler la position 3' de ladite guanosine 2'-O-alkylée 5'-bloquée.

2. Procédé selon la revendication 1, dans lequel la 2,6-diamino-9-(ribofuranosyl)purine est alkylée avec un agent d'alkylation X-L, dans lequel X est R₁-(R₂)ₙ ; R₁ est un groupe alkyle en C₁ à C₂₀, alcényle en C₂ à C₂₀ ou alcynyle en C₂ à C₂₀ ; R₂ est un atome d'halogène, un groupe hydroxyle, thiol, céto, carboxyle, nitro, nitroso, nitrile, trifluorométhyle, trifluorométhoxy, O-alkyle, S-alkyle, NH-alkyle, N-dialkyle, O-aryle, S-aryle, NH-aryle, O-aralkyle, S-aralkyle, NH-aralkyle, amino, N-phtalimido, imidazole, azido, hydrazino, hydroxylamino, isocyanato, sulfoxyde, sulfone, sulfure, disulfure, silyle, aryle, hétérocycle, carbocycle, groupe d'intercalation, molécule rapporteur, conjugué, polyamine, polyamide, polyalkylène glycol ou polyéther ; n est un entier de 0 à 6 ; et L est un groupe partant ; en présence d'un hydrure métallique pour former une 2,6-diamino-9-(ribofuranosyl 2'-O-alkylé)purine.

3. Procédé selon la revendication 1 ou 2, dans lequel ladite étape de blocage ajoute un groupe diméthoxytrityle.

4. Procédé selon la revendication 1 ou 2, dans lequel ladite étape de phosphitylation est conduite avec de la bis-N,N-diisopropylaminocyanoéthylphosphite.

5. Procédé selon la revendication 4, dans lequel ladite étape de phosphitylation est conduite en présence de N,N-diisopropylaminohydrotétrazolide.

6. Procédé selon la revendication 1 ou 2, comprenant en outre le blocage de la fraction N2-amino de ladite guanosine 2'-O-alkylée avant le blocage de ladite fraction 5'-hydroxyle.

7. Procédé selon la revendication 1 ou 2, dans lequel ladite étape de désamination est effectuée en utilisant de l'adénosine désaminase.

8. Procédé de préparation de guanosine 2'-O-substituée comprenant les étapes consistant à :
traiter un 2,6-diaminopurine riboside avec une base ayant un pKb ≥ pKb de NaH et avec un composé répondant à la formule R-L dans laquelle R est un groupe aliphatique ou alicyclique et L est un groupe partant, pour former un 2,6-diaminopurine riboside 2'-O-substitué et un 2,6-diaminopurine riboside 3'-O-substitué dans lesquels ledit substituant est ledit groupe aliphatique ou alicyclique ;
désaminer ledit 2,6-diaminopurine riboside 2'-O-substitué pour donner ladite guanosine 2'-O-substitué ; et
récupérer ladite guanosine 2'-O-substituée.

9. Procédé selon la revendication 8, dans lequel ledit groupe aliphatique ou alicyclique est choisi dans le groupe consistant en une chaîne alkyle, alcényle ou alcynyle droite ou ramifiée en C₁ à C₂₀, ou une chaîne alicyclique en C₁ à C₂₀, respectivement.

10. Procédé selon la revendication 9, dans lequel le substituant de ladite chaîne alkyle, alcényle ou alcynyle droite ou ramifiée substituée en C₁ à C₂₀, ou la chaîne alicyclique substituée en C₁ à C₂₀ est un atome d'halogène, un groupe hydroxyle, thiol, céto, carboxyle, des nitrates, un groupe nitro, nitroso, nitrile, trifluorométhyle, trifluorométhoxy, O-alkyle, S-alkyle, NH-alkyle, N-dialkyle, O-aralkyle, S-aralkyle, NH-aralkyle, amino, azido, hydrazino, hydroxylamino, isocyanato, sulfoxyde, sulfone, sulfure, disulfure, silyle, un groupe hétérocyclique, alicyclique, carbocyclique, des groupes d'intercalation, molécules rapporteurs, conjugués, polyamines, polyamides, polyéthylène glycols et polyéthers.

11. Procédé selon la revendication 8, dans lequel le 2,6-diaminopurine riboside est traité avec un agent d'alkylation X-L, dans lequel X est R₁-(R₂)ₙ ; R₁ est un groupe alkyle en C₁ à C₂₀, alcényle en C₂ à C₂₀ ou alcynyle en C₂ à C₂₀ ; R₂ est un atome d'halogène, un groupe hydroxyle, thiol, céto, carboxyle, nitro, nitroso, nitrile, trifluorométhyle, trifluorométhoxy, O-alkyle, S-alkyle, NH-alkyle, N-dialkyle, O-aryle, S-aryle, NH-aryle, O-aralkyle, S-aralkyle, NH-aralkyle, amino, N-phtalimido, imidazole, azido, hydrazino, hydroxylamino, isocyanato, sulfoxyde, sulfone, sulfure, disulfure, silyle, aryle, hétérocycle, carbocycle, groupe d'intercalation, molécule rapporteur, conjugué, polyamine, polyamide, polyalkylène glycol ou polyéther ; n est un entier de 0 à 6 ; et L est un groupe partant ; pour former un 2,6-diaminopurine riboside 2'-O-substitué et un 2,6-diaminopurine riboside 3'-O-substitué dans lesquels ledit substituant est ledit groupe X.

12. Procédé selon la revendication 8 ou 11, comprenant en outre l'étape consistant à :
séparer ledit 2,6-diaminopurine riboside 2'-O-substitué dudit 2,6-diaminopurine riboside 3'-O-substitué.

13. Procédé selon la revendication 12, dans lequel ledit 2,6-diaminopurine riboside 2'-O-substitué est séparé dudit 2,6-diaminopurine riboside 3'-O-substitué en conduisant ladite désamination dans des conditions telles que la vitesse de désamination dudit 2,6-diaminopurine riboside 2'-O-substitué est accélérée comparée à la vitesse de désamination dudit 2,6-diaminopurine riboside 3'-O-substitué.

14. Procédé selon la revendication 13, dans lequel ladite vitesse accélérée de désamination dudit 2,6-diaminopurine riboside 2'-O-substitué est obtenue par l'utilisation de l'enzyme adénosine désaminase.

15. Procédé selon la revendication 8 ou 11, dans lequel ladite 2,6-diaminopurine 2'-O-substituée est désaminée avec de l'adénosine désaminase.

16. Procédé selon la revendication 8 ou 11, dans lequel ladite base est l'hydrure de sodium.

17. Procédé selon la revendication 8 ou 11, dans lequel ledit 2,6-diaminopurine riboside 2'-O-substitué est physiquement séparé dudit 2,6-diaminopurine riboside 3'-O-substitué par cristallisation ou chromatographie.

18. Procédé de préparation d'une 2'-O-alkyl-guanosine comprenant les étapes consistant à :
traiter un 2,6-diaminopurine riboside avec une base ayant un pKb ≥ pKb de NaH et un composé répondant à la formule R-L dans laquelle R est ledit groupe alkyle et L est un groupe partant, pour former un 2'-O-alkyle-2,6-diaminopurine riboside ;
traiter ledit 2'-O-alkyle-2,6-diaminopurine riboside avec de l'adénosine désaminase ; et
récupérer ladite 2'-O-alkyl-guanosine.

19. Procédé selon la revendication 18, dans lequel ledit groupe alkyle est un alkyle saturé ou insaturé, à chaîne droite ou à chaîne ramifiée en C₁ à C₂₀ ou un groupe alkyle substitué saturé ou insaturé, à chaîne droite ou à chaîne ramifiée en C₁ à C₂₀.

20. Procédé selon la revendication 19, dans lequel ledit groupe alkyle est un groupe alkyle saturé à chaîne droite en C₁ à C₂₀ ou un groupe alkyle substitué saturé à chaîne droite en C₁ à C₂₀.

21. Procédé selon la revendication 19, dans lequel le substituant dudit groupe alkyle substitué saturé ou insaturé, à chaîne droite ou à chaîne ramifiée en C₁ à C₂₀ est un atome d'halogène, un groupe hydroxyle, thiol, céto, carboxyle, des nitrates, un groupe nitro, nitroso, nitrile, trifluorométhyle, trifluorométhoxy, O-alkyle, S-alkyle, NH-alkyle, N-dialkyle, O-aralkyle, S-aralkyle, NH-aralkyle, amino, azido, hydrazino, hydroxylamino, isocyanato, sulfoxyde, sulfone, sulfure, disulfure, silyle, un groupe hétérocyclique, alicyclique, carbocyclique, des groupes d'intercalation, molécules rapporteurs, conjugués, polyamines, polyamides, polyéthylène glycols et polyéthers.

22. Procédé selon la revendication 18, dans lequel le 2,6-diaminopurine riboside est traité avec un agent d'alkylation X-L, dans lequel X est R₁-(R₂)ₙ ; R₁ est un groupe alkyle en C₁ à C₂₀, alcényle en C₂ à C₂₀ ou alcynyle en C₂ à C₂₀ ; R₂ est un atome d'halogène, un groupe hydroxyle, thiol, céto, carboxyle, un groupe nitro, nitroso, nitrile, trifluorométhyle, trifluorométhoxy, O-alkyle, S-alkyle, NH-alkyle, N-dialkyle, O-aryle, S-aryle, NH-aryle, O-aralkyle, S-aralkyle, NH-aralkyle, amino, N-phtalimido, imidazole, azido, hydrazino, hydroxylamino, isocyanato, sulfoxyde, sulfone, sulfure, disulfure, silyle, aryle, hétérocycle, carbocycle, un groupe d'intercalation, molécule rapporteur, conjugué, polyamine, polyamide, polyalkylène glycol ou polyéther ; n est un entier de 0 à 6 ; et L est un groupe partant ; pour former un 2'-O-alkyl-2,6-diaminopurine riboside.

23. Procédé selon la revendication 18 ou 22, dans lequel ladite base est l'hydrure de sodium.

24. Procédé selon la revendication 18 ou 22, comprenant en outre l'étape consistant à :
séparer ledit 2'-O-alkyl-2,6-diaminopurine riboside de produits davantage alkylés résultant dudit traitement dudit 2,6-diaminopurine riboside avec ladite base et ledit composé R-L ou ledit agent d'alkylation X-L, respectivement.

25. Procédé de préparation d'un 3'-O-phosphoramidite de 2'-O-alkyl-guanosine comprenant les étapes consistant à :
traiter un 2,6-diaminopurine riboside avec une base ayant un pKb ≥ pKb de NaH et un composé ayant la formule R-L dans laquelle R est ledit groupe alkyle et L est un groupe partant, pour former un 2'-O-alkyl-2,6-diaminopurine riboside ;
traiter ledit 2'-O-alkyl-2,6-diaminopurine riboside avec de l'adénosine désaminase pour former une 2'-O-alkyl-guanosine ;
protéger la fraction 2-NH₂ de ladite 2'-O-alkyl-guanosine avec un groupe protecteur ;
protéger la fraction 5'-OH de ladite 2'-O-alkyl-guanosine avec un groupe protecteur supplémentaire ;
phosphityler la fraction 3'-OH de ladite 2'-O-alkyl-guanosine protégée ; et
récupérer ledit 3'-O-phosphoramidite de la 2'-O-alkyl-guanosine.

26. Procédé selon la revendication 25, dans lequel ledit 2,6-diaminopurine riboside est traité avec un agent d'alkylation X-L, dans lequel X est R₁-(R₂)ₙ ; R₁ est un groupe alkyle en C₁ à C₂₀, alcényle en C₂ à C₂₀ ou alcynyle en C₂ à C₂₀ ; R₂ est un atome d'halogène, un groupe hydroxyle, thiol, céto, carboxyle, nitro, nitroso, nitrile, trifluorométhyle, trifluorométhoxy, O-alkyle, S-alkyle, NH-alkyle, N-dialkyle, O-aryle, S-aryle, NH-aryle, O-aralkyle, S-aralkyle, NH-aralkyle, amino, N-phtalimido, imidazole, azido, hydrazino, hydroxylamino, isocyanato, sulfoxyde, sulfone, sulfure, disulfure, silyle, aryle, hétérocycle, carbocycle, un groupe d'intercalation, molécule rapporteur, conjugué, polyamine, polyamide, polyalkylène glycol ou polyéther ; n est un entier de 0 à 6 ; et L est un groupe partant ; pour former un 2'-O-alkyl-2,6-diaminopurine riboside.

27. Procédé selon la revendication 25 ou 26, dans lequel ladite 2'-O-alkyl-guanosine protégée est phosphitylée avec de la 2'-cyanométhyl-N,N-diisopropylaminochlorophosphine.

28. Procédé selon la revendication 25 ou 26, dans lequel ladite base est l'hydrure de sodium.
